# FASCICULE DE BREVET EUROPEEN

(11) **EP 0 635 003 B1**
(45) Date de publication et mention de la délivrance du brevet: **17.06.1998**
(21) Numéro de dépôt: 93909005.6
(22) Date de dépôt: 08.04.1993
(51) Int. Cl.: C07D 209/44, A61K 31/40, C07D 409/06

(54) **DERIVES DE PERHYDROISOINDOLE COMME ANTAGONISTES DE LA SUBSTANCE P**
PERHYDROISOINDOLE DERIVATE ALS SUBSTANZ P ANTAGONISTEN
PERHYDROISOINDOLE DERIVATIVES AS P SUBSTANCE ANTAGONISTS

(30) Priorité: 10.04.1992 FR 9204390
(43) Date de publication de la demande: 25.01.1995
(73) Titulaire: RHONE-POULENC RORER S.A., 92160 Antony (FR)
(72) Inventeur: ACHARD, Daniel, F-94320 Thiais (FR); GRISONI, Serge, F-94600 Choisy-le-Roi (FR); MALLERON, Jean-Luc, F-91460 Marcoussis (FR); PEYRONEL, Jean-François, F-91120 Palaiseau (FR); TABART, Michel, F-75013 Paris (FR)
(74) Mandataire: Savina, Jacques
(86) Numéro de dépôt international: FR9300352
(87) Numéro de publication internationale: WO9321155

(56) Documents cités:
- EP-A- 0 429 366
- EP-A- 0 514 273

## Description

La présente invention concerne de nouveaux dérivés du perhydroisoindole de formule générale : 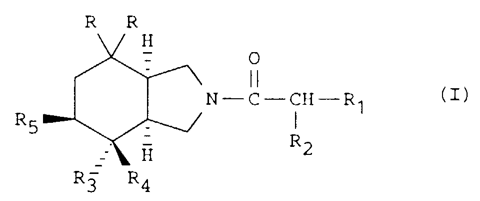 ainsi que leurs sels lorsqu'ils existent, qui antagonisent les effets de la substance P et sont de ce fait particulièrement intéressants dans les domaines thérapeutiques où cette substance est connue pour intervenir.

Dans la demande de brevet européen EP 429 366 ont été décrits des antagonistes de la substance P de structure : 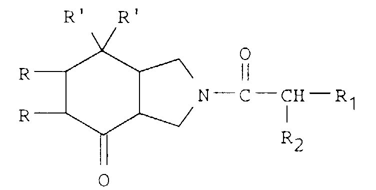 dans laquelle les symboles R sont hydrogène ou forment ensemble une liaison, les symboles R' sont des radicaux phényle éventuellement substitués et les symboles R₁ et R₂ représentent diverses substitutions. cependant ces dérivés de perhydroisoindolone se sont montrés principalement actifs dans des tests de binding utilisant des homogénats de cerveau de rat et manifestent moins d'activité dans des tests de binding utilisant des cellules humaines lymphoblastiques en culture.

Dans le brevet américain 4 042 707 avaient été décrits des produits dérivés de l'isoindole de formule générale: 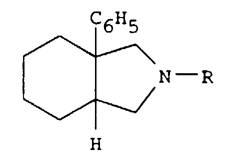 ayant une activité opiacée. Ces produits n'ont pas d'activité vis à vis de la substance P.

Jusqu'à présent, malgré les recherches mises en oeuvre et malgré l'intérêt suscité [M.R. Hanley, TINS, (5) 139 (1982)], il n'avait pratiquement pas été découvert de produit agissant spécifiquement sur la substance P et ayant une structure non peptidique, c'est pourquoi les dérivés de l'isoindole de formule générale (I) présentent un intérêt considérable.

Dans la formule générale (I) :
- les symboles R sont identiques et représentent des radicaux phényle éventuellement substitués par un atome d'halogène ou par un radical méthyle en position 2 ou 3,
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle, indényle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle ou alcoyloxy,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino,
- le symbole R₃ représente un radical phényle éventuellement substitué en position -2 par un radical alcoyle ou alcoyloxy contenant 1 ou 2 atomes de carbone, et
- le symbole R₄ représente un atome de fluor ou un radical hydroxy, et le symbole R₅ représente un atome d'hydrogène ou bien
- les symboles R₄ et R₅ représentent des radicaux hydroxy, ou bien
- le symbole R₄ forme avec R₅ une liaison.

Il est entendu que les radicaux alcoyle ou acyle cités ci-dessus contiennent (sauf mention spéciale) 1 à 4 atomes de carbone en chaîne droite ou ramifiée.

Lorsque R porte un substituant halogène, ce dernier peut être choisi parmi le chlore ou le fluor.

Lorsque R₁ contient un atome d'halogène, ce dernier peut être choisi parmi le chlore, le brome, le fluor ou l'iode.

Lorsque R₁ représente un radical hétérocyclyle mono ou polycyclique, saturé ou insaturé, à titre d'exemple il peut être choisi parmi thiényle, furyle, pyridyle, dithiinyle, indolyle, isoindolyle, benzothiényle, thiazolyle, isothiazolyle, oxazolyle, imidazolyle, pyrrolyle, triazolyle, thiadiazolyle, quinolyle, isoquinolyle, ou naphtyridinyle.

Lorsque R₁ représente phényle substitué par une chaîne portant un hétérocycle, ce dernier peut être choisi parmi pyrrolidinyle, morpholino, pipéridinyle, tétrahydropyridinyle, pipérazinyle, thiomorpholino.

Plus spécifiquement, les dérivés de perhydroisoindole pour lesquels R₄ est hydroxy ou fluor et R₅ est hydrogène, de forme (3aS,4S,7aS) à l'état pur, ou sous forme de mélange racémique (3aRS,4RS,7aRS), les dérivés de perhydroisoindole pour lesquels R₄ et R₅ sont hydroxy, de forme (3aS,4S,5S,7aS) à l'état pur, ou sous forme de mélange racémique (3aRS,4RS,5RS,7aRS), les dérivés de perhydroisoindole pour lesquels R₄ forme avec R₅ une liaison, de forme (3aS,7aR) à l'état pur, ou sous forme de mélange racémique (3aRS,7aSR) entrent dans le cadre de la présente invention. De plus, lorsque le symbole R₂ est autre que l'atome d'hydrogène, la chaîne substituée sur l'isoindole présente un centre chiral, il est entendu que les formes stéréoisomères (R) ou (S) et leurs mélanges font partie de la présente invention.

Selon l'invention les dérivés du perhydroisoindole de formule générale (I) peuvent être obtenus par action de l'acide de formule générale : 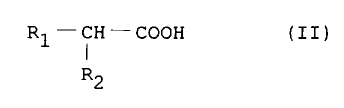 ou d'un dérivé réactif de cet acide, dans lequel R₁ et R₂ sont définis comme précédemment, sur un dérivé de l'isoindole de formule générale : 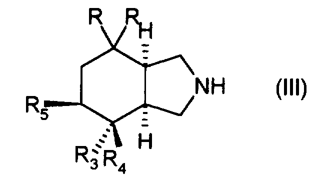 dans laquelle les symboles R, R₃, R₄ et R₅ sont définis comme précédemment, puis éventuellement transformation du produit obtenu pour lequel R₄ est un radical hydroxy et R₅ est un atome d'hydrogène en un produit pour lequel R₄ est un atome de fluor et R₅ est un atome d'hydrogène ou en un produit pour lequel R₄ et R₅ forment ensemble une liaison.

Il est entendu que, les radicaux amino, alcoylamino ou carboxy contenus dans R₁ et/ou R₂ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

A titre d'exemple,
- les groupements amino ou alcoylamino peuvent être protégés par des radicaux methoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque R₂ représente un radical hydroxy, il est préférable de protéger préalablement ce radical. La protection s'effectue par exemple par un radical acétyle, trialcoylsilyle, benzyle, sous forme d'un carbonate par un radical -COORa dans lequel Ra est un radical alcoyle ou benzyle ou sous forme de cétone.

Lorsque l'on effectue la condensation d'un dérivé réactif de l'acide de formule générale (II), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement a une température comprise entre -40 et +40°C, dans un solvant organique tel qu'un solvant chloré (dichlorométhane, dichloréthane, chloroforme par exemple), un hydrocarbure (toluène par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le NN'-carbonyldiimidazole ou l'ethoxy-2 éthoxycarbonyl-1 dihydro-1,2 quinoléine ou bien en milieu hydroorganique, en présence d'un agent alcalin de condensation comme le bicarbonate de sodium.

Dans l'alternative où l'on a obtenu un dérivé de perhydroisoindole de formule générale (I) pour lequel R₄ est un radical hydroxy et R₅ est un atome d'hydrogène et où l'on veut obtenir un dérivé de perhydroisoindole pour lequel R₄ est un atome de fluor et R₅ est un atome d'hydrogène, on opère par fluoration du dérivé précédemment obtenu.

La réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme le tétrafluorure de sélenium (J. Am. Chem. Soc., 96,925 (1974) ou comme le tétrafluorophénylphosphorane (Tet. Let., 907 (1973), en opérant dans un solvant organique tel qu'un solvant chloré (dichlorométhane, dichloréthane par exemple) à une température comprise entre -30 et 30°C.

Dans l'alternative où l'on a obtenu un dérivé de perhydroisoindole de formule générale (I) pour lequel R₄ est un radical hydroxy et R₅ est un atome d'hydrogène et où l'on veut obtenir un dérivé de perhydroisoindole pour lequel R₄ et R₅ forment ensemble une liaison, on opère par toute méthode connue de deshydratation des alcools qui n'altère pas le reste de la molécule. Notamment on effectue la deshydratation en milieu acide par exemple par action d'un acide sulfonique (acide p.toluènesulfonique...), de l'acide sulfurique, de l'acide phosphorique, du pentaoxyde de phosphore ou de l'oxyde d'aluminium ou par action d'un mélange acide chlorhydrique-acide acétique ou acide bromhydrique-acide acétique, à une température comprise entre 25°C et la température de reflux du mélange réactionnel.

Selon l'invention les dérivés de l'isoindole de formule générale (I) pour lesquels R₅ est autre que le radical hydroxy, peuvent également être obtenus par action d'un composé organométallique de formule générale :

R₃-M (IV)

dans laquelle R₃ est défini comme précédemment, et M représente le lithium, un radical MgX ou CeX₂ pour lequel X est un atome d'halogène, sur un dérivé de perhydroisoindolone de formule générale : 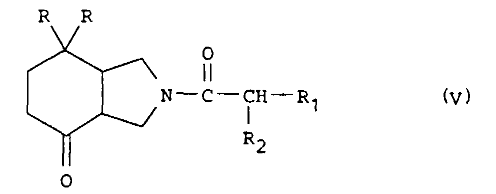 dans laquelle R, R₁ et R₂ sont définis comme précédemment, puis transforme éventuellement l'alcool obtenu de formule générale (I) en un dérivé de perhydroisoindole pour lequel R₄ est un atome de fluor et R₅ est un atome d'hydrogène ou en un dérivé de perhydroisoindole pour lequel R₄ et R₅ forment ensemble une liaison.

La réaction s'effectue en milieu anhydre, dans les conditions habituelles de réaction des composés organométalliques sur une cétone, qui n'affectent pas le reste de la molécule. Notamment on opère dans un éther (par exemple le tétrahydrofuranne ou l'éther éthylique) éventuellement en présence de chlorure de cérium anhydre à une température comprise entre -78 et 30°C.

Les opérations subséquentes de transformation en un dérivé de formule générale (I) pour lequel R₄ est un atome de fluor et R₅ est hydrogène ou pour lequel R₄ et R₅ forment ensemble une liaison, s'effectuent dans les conditions décrites précédemment.

Les acides de formule générale (Il) peuvent être préparés selon les méthodes décrites ci-après dans les exemples, selon les méthodes décrites dans la demande de brevet EP 429 366 ou par analogie avec ces méthodes.

Le dérivé de perhydroisoindole de formule générale (III) pour lequel R₄ et R₅ forment ensemble une liaison peuvent être obtenus par deshydratation du dérivé correspondant de perhydroisoindole pour lequel R₄ est un radical hydroxy et R₅ est un atome d'hydrogène.

La réaction s'effectue dans les conditions décrites précédemment pour la préparation des dérivés de formule générale (I) dans laquelle R₄ et R₅ forment ensemble une liaison à partir du dérivé correspondant de perhydroisoindole pour lequel R₄ est un radical hydroxy et R₅ est un atome d'hydrogène.

Le dérivé de l'isoindole de formule générale (III) pour lequel R₄ est un atome de fluor et R₅ est un atome d'hydrogène peut être préparé par fluoration d'un dérivé de l'isoindole de formule générale : 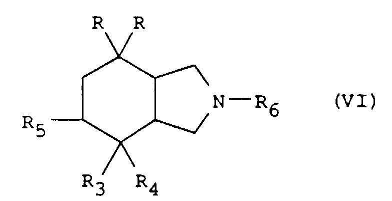 dans laquelle R et R₃ sont définis comme précédemment, R₆ est un radical protecteur, R₄ est un radical hydroxy et R₅ est un atome d'hydrogène, puis élimination du radical protecteur R₆.

Le radical protecteur R₆ peut être tout groupement protecteur d'amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloracétyle, trifluoracétyle, vinyloxycarbonyle, phénoxycarbonyle, chloro-1 éthoxycarbonyle ou chlorocarbonyle.

Lorsque l'on veut obtenir un dérivé fluoré du perhydroisoindole de formule générale (III) la fluoration s'effectue dans les conditions décrites précédemment pour la fluoration d'un dérivé de formule générale (I) dans laquelle R₄ est hydroxy, à une température comprise entre -30 et +30°C.

L'élimination subséquente du radical protecteur R₆ s'effectue selon les méthodes habituelles. Notamment selon les méthodes décrites par T.W. Greene, par A. Wiley ou par Mc Omie dans les références citées précédemment.

Le dérivé de perhydroisoindole de formule générale (III) ou (VI) pour lequel R₄ est un radical hydroxy et R₅ est un atome d'hydrogène ou un radical hydroxy, peut être obtenu par action d'un composé organométallique de formule générale (IV) sur le dérivé correspondant de perhydroisoindolone de formule générale : 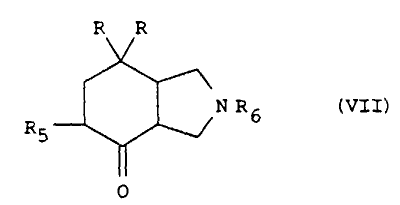 dans laquelle R et R₆ sont définis comme précédemment, et R₅ est un atome d'hydrogène ou un radical hydroxy éventuellement protégé, puis libération du radical protecteur de R₅ et éventuellement élimination du radical protecteur R₆.

La réaction s'effectue dans des conditions analogues à celles décrites pour l'obtention du perhydroisoindole de formule générale (I) à partir de la perhydroisoindolone correspondante. Il est entendu que selon la nature du radical protecteur du radical R₅, ce dernier peut être éliminé simultanément à la réaction.

Le dérivé de perhydroisoindole de formule générale (VI) peut être préparé par protection de l'amino du dérivé correspondant de formule générale (III).

La protection s'effectue selon les méthodes habituelles. Notamment selon les références citées précédemment.

Le dérivé de la perhydroisoindolone de formule générale (VII) pour laquelle R₅ est un atome d'hydrogène peut être préparé par analogie avec la méthode décrite dans la demande de brevet européen EP 429 366. Le dérivé de la perhydroisoindolone de formule générale (VII) pour lequel R₅ est un radical hydroxy préalablement protégé peut être aussi préparé par analogie avec cette méthode, ou comme décrit ci-après dans les exemples.

La préparation du dérivé de la perhydroisoindolone de formule générale (V) s'effectue par analogie avec la méthode décrite dans la demande de brevet européen EP 429 366.

Il est entendu que les dérivés de perhydroisoindole de formule générale (I), (III), (V), (VI) et (VII) présentent plusieurs formes stéréoisomères. Lorsque l'on veut obtenir un produit de formule générale (I) de forme (3aS,7aS), la séparation des formes isomères est mise en oeuvre de préférence au niveau du dérivé de formule générale (VII) ou au niveau d'un autre intermédiaire portant un radical oxo en position -4. Elle peut aussi être mise en oeuvre au niveau du dérivé de formule générale (III). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique ou ditoluoyltartrique, puis séparation des isomères par cristallisation. L'isomère recherché est libéré de son sel en milieu basique.

Les nouveaux dérivés de l'isoindole de formule générale (I) peuvent être purifiés le cas échéant par des méthodes physiques telles que la cristallisation ou la chromatographie.

Le cas échéant, les nouveaux dérivés de formule générale (I) pour lesquels les symboles R₁ et/ou R₂ contiennent des substituants amino ou alcoylamino, peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindole de formule générale (I) peuvent aussi, le cas échéant, lorsque R₂ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhyldrylamine, quinine, choline, arginine, lysine, leucine, dibenzylamine).

Les nouveaux dérivés de l'isoindole selon la présente invention qui antagonisent les effets de la substance P peuvent trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 10 et 1000 nM selon les techniques adaptées de D.G. Payan et coll., J. of immunology, 133(6), 3260-5 (1984) : Stereospecific receptors for substance P on cultured human IM-9 lymphoblasts et de Mc Pherson et coll., J. Pharmacol. Meth., 14, 213 (1985) : Analysis of radioligand binding experiments.

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés manifestent un antagonisme des contractions de l'iléon de cobaye induites par la substance P ou des contractions de l'iléon de cobaye induites par le septide, à des concentrations de 6 à 1000 nM.

La substance P est connue pour être impliquée dans un certain nombre de domaines pathologiques :
- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiocascular Pharmacology, 10 (suppl. 12), 5172 (1987).

L'étude de certains dérivés de l'isoindole de formule générale (I) dans la technique de A. Saria et coll., Arch. Pharmacol.,324, 212-218 (1983) adaptée au cobaye a permis de mettre en évidence un effet inhibiteur de l'augmentation de la perméabilité capillaire entraîné par le septide (agoniste de la substance P), ce qui témoigne d'une activité anti-inflammatoire :

| Produit étudié | DE₅₀ |
|---|---|
| Exemple 1 | 0,04 mg/kg i.v. |
| | 3,5 mg/kg p.o. |

L'injection de substance P chez l'animal provoque une hypotension. Les produits étudiés dans la technique de C.A. Maggi et coll., J. Auton. Pharmac., 7, 11-32 (1987) manifestent un effet antagoniste vis-à-vis de cette hypotension, chez le cobaye. On détermine la DE₅₀ dose qui diminue de 50% l'hypotension induite par une injection i.v. de 250 ng/kg de substance P.

| Produit de formule générale (I) | DE₅₀ mg/kg i.v. |
|---|---|
| Exemple 1 | 0,15 |

L'injection de substance P provoque chez l'animal un bronchospasme. La bronchoconstriction induite in vivo chez le cobaye par l'injection de substance P ou d'un agoniste sélectif de la substance P : [Pro⁹] substance P, est étudiée selon la technique de H. Konzett et R. Rosseler, Archiv. Exp. Path. Pharmak., 195, 71-74 (1940). Cette bronchoconstriction est inhibée par l'injection d'un produit selon l'invention, ce qui témoigne d'une activité anti-asthmatique. On détermine la DE₅₀, dose qui diminue de 50% le bronchospasme induit par 3 µg/kg i.v. de [Pro⁹] substance P. Dans cette technique la DE₅₀ du produit de l'exemple 1 est de 0,7 mg/kg i.v..

Par ailleurs, dans le test de douleur au formol chez le cobaye, le produit de l'exemple 1 administré par voie orale 1 heure avant le test, manifeste une DE₅₀ de 11 mg/kg.

Enfin, les dérivés de l'isoindole selon la présente invention ne présentent pas de toxicité, ils se sont montrés atoxiques chez la souris par voie intra-veineuse à la dose de de 10 mg/kg ou par voie sous cutanée à la dose de 40 mg/kg par voie sous-cutanée.

D'un intérêt particulier sont les produits de formule générale (I) pour lesquels :
- les symboles R sont identiques et représentent des radicaux phényle,
- le symbole R₁ représente un radical phényle éventuellement substitué par un ou plusieurs radicaux hydroxy, alcoyloxy, dialcoylamino , naphtyle, indényle ou hétérocyclyle mono ou polycyclique choisi parmi thiényle, indolyle, benzothiényle et éventuellement substitué par un atome d'halogène ou par un radical alcoyle ou alcoyloxy,
- le symbole R₂ représente un atome d'hydrogène ou un radical hydroxy, alcoyle ou amino,
- le symbole R₃ représente un radical phényle éventuellement substitué en position -2 par un radical alcoyle ou alcoyloxy contenant 1 ou 2 atomes de carbone, et
- le symbole R₄ représente un atome de fluor ou un radical hydroxy, et le symbole R₅ représente un atome d'hydrogène ou bien
- les symboles R₄ et R₅ représentent des radicaux hydroxy, ou bien
- le symbole R₄ forme avec R₅ une liaison.
Et parmi ces produits plus spécialement les :
diphényl-7,7 (méthoxy-2 phényl)-4 [(méthoxy-2 phényl)-2 propionyl]-2 perhydroisoindolol-4;
diphényl-7,7 (méthoxy-2 phényl)-4 [(méthoxy-2 phényl)-2 propionyl]-2 perhydroisoindolediol-4,5;
diphényl-7,7 (méthoxy-2 phényl)-4 [(hydroxy-2 phényl)-2 acétyl]-2 perhydroisoindolol-4;
diphényl-7,7 (méthoxy-2 phényl)-4 (indolyl-3 acétyl)-2 perhydroisoindolediol-4,5;
diphényl-7,7 (méthoxy-2 phényl)-4 [(méthoxy-2 phényl)-acétyl]-2 perhydroisoindolol-4;
diphényl-7,7 (méthoxy-2 phényl)-4 [(hydroxy-2 phényl)-2 propionyl]-2 perhydroisoindolol-4;
sous forme racémique, sous les formes stéréoisomères citées précédemment ou sous forme de mélange.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

### Exemple 1

A une suspension de 0,8 g de chlorhydrate de diphényl-7,7 (méthoxy-2 phényl)-4 perhydroisoindolol-4-(3aS,4S,7aS) dans 60 cm³ de dichlorométhane sec on ajoute 0,025 g d'hydroxy-1 benzotriazole, 0,38 g d'acide (méthoxy-2 phényl)-2 propionique-(S), 0,32 cm³ de diisopropyléthylamine, puis on refroidit cette solution à +5°C et on ajoute rapidement une suspension de 0,43 g de chlorhydrate de (diméthylamino-3 propyl)-1 éthyl-3 carbodiimide dans 10 cm³ de dichlorométhane sec. Le mélange réactionnel est agité 2 heures à +5°C, 2 heures à température ambiante, lavé par 20 cm ³ d'eau, puis lavé par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 2,8 cm, hauteur 20 cm), en éluant sous une pression de 0,5 bar d'azote par un mélange de cyclohexane et d'acétate d'éthyle (80/20 en volumes) et en recueillant des fractions de 25 cm^{3.} Les fractions 9 à 15 sont réunies puis concentrées à sec sous pression réduite (2,7 kPa). Le résidu est cristallisé dans un mélange d'acétonitrile et d'oxyde de diisopropyle. On obtient 0,17 g de diphényl-7,7 (méthoxy-2 phényl)-4 [(méthoxy-2 phényl)-2 propionyl-(S)]-2 perhydroisoindolol-4(3aS,4S,7aS) sous la forme de cristaux blancs fondant à 244°C.

Le chlorhydrate de diphényl-7,7 (méthoxy-2 phényl)-4 perhydroisoindolol-4-(3aS,4S,7aS) peut être préparé de la manière suivante :

A une solution de 7,63 g de diphényl-7,7 (méthoxy-2 phényl)-4 tertbutoxycarbonyl-2 perhydroisoindolol-4-(3aS,4S,7aS) dans 66 cm ³ de dioxanne on ajoute à température ambiante une solution de 100 cm³ de dioxanne chlorhydrique 5,2 N. Le mélange réactionnel est agité 1 heure à cette température, puis concentré à sec sous pression réduite (2,7 kPa). Le résidu est lavé par de l'acétonitrile, essoré puis séché. On obtient 4,88 g de chlorhydrate de diphényl-7,7 (méthoxy-2 phényl)-4 perhydroisoindolol-4-(3aS,4S,7aS) sous la forme de cristaux blancs fondant à 271°C (bloc Maquenne).

Le diphényl-7,7 (méthoxy-2 phényl)-4 tert-butoxycarbonyl-2 perhydroisoindolol-4-(3aS,4S,7aS) peut être préparé de la manière suivante :

A une suspension de 20 g de diphényl-7,7 tert-butoxycarbonyl-2 perhydroisoindolone-4-(3aS,7aS) et de 31,6 g de chlorure de cérium anhydre dans 250 cm³ de tétrahydrofuranne sec, on ajoute goutte à goutte à température ambiante sous agitation une suspension de bromure de méthoxy-2 phénylmagnésium (préparée à partir de 75,3 g de bromo-2 anisole et de 9,8 g de magnésium) dans 100 cm³ de tétrahydrofuranne sec. Le mélange réactionnel est agité à température ambiante pendant 24 heures, traité par 400 cm³ d'une solution aqueuse saturée en chlorure d'ammonium, dilué par 200 cm³ d'acétate d'éthyle, lavé par 300 cm³ d'eau (deux fois), puis par 300 cm³ d' une solution aqueuse saturée en chlorure de sodium, séché sur sulfate de magnésium, filtré et concentré à sec sous pression réduite (2,7 kPa). Le résidu est chromatographié sur colonne de gel de silice Merck (granulométrie 0,04-0,06 mm, diamètre 5,8 cm, hauteur 26,5 cm), en éluant sous une pression de 0,5 bar d'azote par un mélange de cyclohexane et d'acétate d'éthyle (80/20 en volumes) et en recueillant des fractions de 100 cm³. Les fractions 9 à 29 sont réunies et concentrées à sec sous pression réduite (2,7 kPa). On obtient 17,82 g de diphényl-7,7 (méthoxy-2 phényl)-4 tert-butoxycarbonyl-2 perhydroisoindolol-4-(3aS,4S,7aS) sous forme d'une meringue blanche.

Spectre RMN du proton (DMSO d₆): 1,36 (s, 9H, -C(CH₃)₃); 1,54 (dmt, J=14, 1H, H équatorial du -CH₂- en 5); 2,3 (dmt, J=14, 1H, H équatorial du -CH₂- en 6); 2,34 (td, J=14 et 2,5, 1H, H axial du -CH₂- en 5); 3,07(td, J=14 et 2,5, H axial du -CH₂-en 6); 3,49(s, 3H, -OCH₃); 2,6 à 3,6(mt, autre -CH₂- et -CH); 6,85 à 7,7 (mt, 14H, aromatiques).

La diphényl-7,7 tert-butoxycarbonyl-2 perhydroisoindolone-4-(3aS,7aS) peut être obtenue de la manière suivante :

A une suspension de 80 g de chlorhydrate de diphényl-7,7 perhydroisoindolone-4-(3aS,7aS) dans 400 cm³ de dichlorométhane sec on ajoute à température ambiante sous agitation 34,3 cm³ de triéthylamine, 58,6 g de di-tert-butyl dicarbonate, puis 2,98 g de diméthylamino-4 pyridine. Le mélange réactionnel est agité à température ambiante pendant 24 heures, lavé par 100 cm³ d'une solution aqueuse d'acide citrique, puis par 100 cm d'une solution aqueuse d'hydrogénocarbonate de sodium, puis par 100 cm³ d'une solution aqueuse saturée en chlorure de sodium, séché sur sulfate de magnésium, et concentré à sec sous pression réduite (2,7 kPa). On obtient 106,5 g de diphényl-7,7 tert-butoxycarbonyl-2 perhydroisoindolone-4(3aS,7aS) sous la forme d'une meringue orange.

Spectre RMN du proton (DMSO d₆): 1,4(s, 9H, -C(CH₃)₃); 2,11(td, J=15 et 7,5, 1H, H axial du -CH₂-en 5); 2,3(dt, J=15 et 3,5, 1H, H équatorial du -CH₂- en 5); 2,75 à 2,9 (mt, 4H, -CH₂- en 6 et -CH₂- en 1); 3,26 (dd, J=7,5 et 7, 1H, -CH en 3a); 3,35 (dd, J=11 et 7, 1H, 1H du -CH₂- en 3); 3,97 (mt, 1H, -CH en 7a); 4,1(d, J-11, 1H, l'autre H du -CH₂- en 3); 7,1 à 7,7 (mt, 10H, aromatiques).

Le chlorhyrate de diphényl-7,7 perhydroisoindolone-4-(3aS,7aS) peut être obtenu de la manière suivante :

A une suspension de 20 g de chlorhydrate de diphényl-7,7 perhydroisoindolone-4-(3aRS,7aRS) dans 250 cm³ d'acétate d'éthyle, on ajoute lentement, sous agitation, 50 cm³ de soude aqueuse 4N; l'agitation est poursuivie jusqu'à disparition du produit de départ. La solution organique est lavée par 100 cm³ d'eau distillée, par 100 cm³ d'une solution saturée en chlorure de sodium, séchée sur sulfate de magnésium et filtrée. A la solution ainsi obtenue, on ajoute sous agitation une solution de 9,3 g d'acide D-(-) mandélique dans 50 cm³ d'acétate d'éthyle. Les cristaux formés sont filtrés essorés, lavés par 50 cm³ d'acétate d'éthyle (deux fois) et séchés. Les cristaux sont repris par une solution de 220 cm³ d'acétonitrile et de 60 cm³ d'eau distillée, le mélange est porté au reflux sous agitation pendant 15 minutes; les cristaux formés sont filtrés, et à nouveau cristallisés dans un mélange de 100 cm³ d'acétonitrile et de 35 cm³ d'eau distillée. On obtient 6,4 g de D-mandélate de diphényl-7,7 perhydroisoindolone-4-(3aS,7aS).

A 6,4 g de D-mandélate de diphényl-7,7 perhydroisoindolone-4(3aS,7aS) en solution dans 100 cm³ d'acétate d'éthyle on ajoute 50 cm³ de soude aqueuse 1N; le mélange réactionnel est agité à température ambiante jusqu'à disparition du produit de départ; la solution organique est lavée par 50 cm³ d'eau distillée, par 50 cm³ d'une solution aqueuse saturée en chlorure de sodium, séchée sur sulfate de magnésium et filtrée; elle est acidifiée sous agitation par addition de 2 cm³ d'une solution 9N d'acide chlorhydrique dans l'éthanol; les cristaux obtenus sont essorés, lavés par de l'acétate d'éthyle, puis par de l'oxyde d'isopropyle et séchés. On obtient 4,24 g de chlorhydrate de diphényl-7,7 perhydroisoindolone-4-(3aS,7aS) sous la forme de cristaux blancs fondant à 270°C avec décomposition.

L'acide (méthoxy-2 phényl)-2 propionique-(S) peut être préparé, par analogie avec les méthodes décrites par D.A Evans et coll., Tetrahedron, 44, 5525, (1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. [α]_{D}²⁰ = +84,6° (c=1; CHCl₃).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)propionyl]-3 oxazolidinone-2-(4S,5S) sous la forme d'un solide blanc.

La méthyl-4 phényl-5 (méthoxy-2 phénylacétyl)-3 oxazolidinone-2(4S,5S) peut être obtenue de la manière suivante :

A une suspension de 1,89 d'hydrure de sodium (dispersion à 80% dans la vaseline) dans 200 cm³ de tétrahydrofuranne sec on ajoute à température ambiante 9,38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35,27 cm³ de butyllithium 1,6 M dans l'hexane à une solution refroidie à -78°C de 10 g de méthyl-4 phényl-5 oxazolidinone-2-(4S,5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle; aprés décantation la phase organique est lavée deux fois par 100 cm³ d'eau, puis deux fois par 100 cm³ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 cm³ . Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

### Exemple 2

En opérant selon le mode opératoire de l'exemple 22 ci-après à partir de 0,68 g de chlorhydrate de diphényl-7,7 (méthyl-2 phényl)-4 perhydroisoindolol-4-(3aS,4S,7aS) et de C,28 cm³ de chlorure de phénylacétyle on obtient 0,4 g de diphényl-7,7 (méthyl-2 phényl)-4 phénylacétyl-2 perhydroisoindolol-4-(3aS,4S,7aS) sous la forme de cristaux blancs fondant à 208°C.

Le chlorhydrate de diphényl-7,7 (méthyl-2 phényl)-4 perhydroisoindolol-4-(3aS,4S,7aS) peut être préparé de la manière suivante:

En opérant selon l'exemple 1 à partir de 1,2 g de diphényl-7,7 (méthyl-2 phényl)-4 tert-butoxycarbonyl-2 perhydroisoindolol-4(3aS,4S,7aS) on obtient 0,68 g de chlorhydrate de diphényl-7,7 (méthyl-2 phényl)-4 perhydroisoindolol-4-(3aS,4S,7aS).

Spectre infra-rouge (KBr), bandes caractéristiques (cm⁻¹):
3325, 3100-3000, 3000-2850, 3000-2300, 1600, 1585, 1560, 1495, 1445, 750, 700.

Le diphényl-7,7 (méthyl-2 phényl)-4 tert-butoxycarbonyl-2 perhydroisoindolol-4-(3aS,4S,7aS) peut être préparé de la manière suivante :

En opérant selon le mode opératoire de l'exemple 1 à partir de 3 g de diphényl-7,7 tert-butoxycarbonyl-2 perhydroisoindolone-4-(3aS,7aS) et d'une suspension de bromure de méthyl-2 phénylmagnésium (préparée à partir de 4,6 cm³ de bromo-2 toluène et de 0,93 g de magnésium dans 15 cm³ de tétrahydrofuranne anhydre) on obtient 1,5 g de diphényl-7,7 (méthyl-2 phényl)-4 tert-butoxycarbonyl-2 perhydroisoindolol-4(3aS,4S,7aS) sous forme d'une huile utilisée telle quelle dans l'essai suivant.

Exemple 3 A une suspension de 1,1 g de chlorhydrate de diphényl7,7 (méthoxy-2 phényl)-4 perhydroisoindolol-4-(3aRS,4RS,7aRS) dans 25 cm de dichlorométhane sec on ajoute à température ambiante 0,35 cm³ de triéthylamine, puis 0,33 cm³ de chlorure de phénylacétyle. Le mélange réactionnel est agité 24 heures à cette température, dilué par 200 cm³ de dichlorométhane, lavé par 100 cm³ d'une solution saturée en hydrogénocarbonate de sodium, par 100 cm³ d'eau (deux fois), puis par une solution aqueuse saturée en chlorure de sodium, séché sur sulfate de magnésium, filtré et concentré à sec sous pression réduite (2,7 kPa).Le résidu est chromatographié sur colonne de gel de silice Merck (granulométrie 0,04-0,06 mm, diamètre 2 cm, hauteur 22 cm), en éluant sous une pression d'azote de 50 KPa par un mélange de cyclohexane et d'acétate d'éthyle (55/45 en volumes) et en recueillant des fractions de 20 cm^{3.} Les fractions 11 à 18 sont réunies et concentrées à sec sous pression réduite (2,7 kPa). Le résidu obtenu est cristallisé dans 70 cm³ d'acétonitrile, les cristaux sont essorés et séchés. On obtient 0,5 g de diphényl-7,7 (méthoxy-2 phényl)-4 phénylacétyl-2 perhydroisoindolol-4(3aRS,4RS,7aRS) sous la forme d'un solide blanc.

Spectre RMN du proton (DMSO d₆): 1,5 (dmt, J=14, 1H, 1H équatorial du -CH₂- en 5); 2,26 (dmt, J=14, 1H, H équatorial du -CH₂- en 6); 2,31 (td, J=14 et 3, 1H, H axial du -CH₂- en 5); 2,85 (mt, 1H,-CH<en 3a); 3,02 (td, J=14 et 2,5, 1H, H axial du -CH₂- en 6); 3,2 à 3,6 (mf, - CH₂- et CH<); 3,44 (s, 3H, -OCH₃); 6,8 à 7,6 (mt, 19 H aromatiques).

Spectre infra-rouge (KBr), bandes caractéristiques (cm⁻¹) : 3100-3000, 3000-2850, 2840, 1640, 1600, 1580, 1495, 1450, 1245, 1030,750, 720, 700.

Le chlorhydrate de diphényl-7,7 (méthoxy-2 phényl)-4 perhydroisoindolol-4-(3aRS,4RS,7aRS) peut être préparé de la manière suivante :

En opérant selon le mode opératoire de l'exemple 1 à partir de 2,7 g de diphényl-7,7 (méthoxy-2 phényl)-4 tert-butoxycarbonyl-2 perhydroisoindolol-4-(3aRS,4RS,7aRS) on obtient 1,77 g de chlorhydrate de diphényl-7,7 (méthoxy-2 phényl)-4 perhydroisoindolol-4- (3aRS,4RS, 7aRS) sous la forme d'un solide blanc.

Spectre RMN du proton (DMSO d₆): 1,55 (d large, J=14, 1H, H équatorial du -CH₂- en 5); 2,34 (td, J=14 et 2,5, 1H, H axial du -CH₂- en 5); 2,37 (d large, J=14, 1H, H équatorial du -CH₂- en 6); 2,52 (mt, 1H du -CH₂- en 1); 2,93 (td, J=14 et 2,5; 1H, H axial du -CH₂- en 6); 3 à 3,3 (mt, 3H, -CH₂-en 3 et l'autre H du -CH₂- en 1); 3,42 (s, 3H,OCH₃-); 3,4 à 3,7 (mt, 2H, -CH< en 3a et 7a); 5,3 (mf étalé, 1H, - OH); 6,8 à 7,7 (mt, 14H, aromatiques).

Le diphényl-7,7 (méthoxy-2 phényl)-4 tert-butoxycarbonyl-2 perhydroisoindolol-4-(3aRS,4RS,7aRS) peut être obtenu de la manière suivante :

En opérant selon le mode opératoire de l'exemple 1 à partir de 2,75 g de diphényl-7,7 tert-butoxycarbonyl-2 perhydroisoindolone-4-(3aRS, 7aRS), de 1,73 g de chlorure de cérium anhydre et d'une suspension de bromure de méthoxy-2 phénylmagnésium (obtenue à partir de 6,57 g de bromo-2 anisole et de 0,84 g de magnésium) on obtient 2,72 g de diphényl-7,7 (méthoxy-2 phényl)-4 tert-butoxycarbonyl-2 perhydroisoindolol-4-(3aRS,4RS,7aRS) sous forme d'une meringue blanche.

Spectre RMN du proton (DMSO d₆): A température ambiante, on observe le mélange des rotamères: 1,3 et 1,35 (mt, 1H, 1H du -CH₂- en 5); 2,15 à 2,4 (mt, 2H, l'autre H du -CH₂- en 5 et 1H du -CH₂- en 6); 2,5 à 3,6 (mt, -CH₂- et -CH<); 3,35 et 3,39 (2s, 3H, -OCH₃); 4,68 et 4,72 (2s, 1H, -OH); 6,8 à 7,7 (mt, 14H aromatiques).

La diphényl-7,7 tert-butoxycarbonyl-2 perhydroisoindolone-4(3aRS,7aRS) peut être préparée de la manière suivante :

A une suspension de 10 g de chlorhydrate de diphényl-7,7 perhydroisoindolone-4-(3aRS,7aRS) dans 50 cm³ de dichlorométhane sec on ajoute à température ambiante sous agitation 4,3 cm³ de triéthylamine, 7,4 g de di-tert-butyl dicarbonate, puis 0,37 g de diméthylamino-4 pyridine. Le mélange réactionnel est agité à température ambiante pendant 24 heures, lavé par 150 cm³ d'une solution aqueuse d'acide citrique, puis par 100 cm³ d'une solution aqueuse d'hydrogénocarbonate de sodium, puis par 100 cm³ d'une solution aqueuse saturée en chlorure de sodium, séché sur sulfate de magnésium, et concentré à sec sous pression réduite (2,7 kPa). On obtient 11 g de diphényl-7,7 tertbutoxycarbonyl-2 perhydroisoindolone-4-(3aRS,7aRS) sous la forme d'une meringue crème.

Spectre RMN du proton (DMSO d₆): 1,38 (s, 9H, -C(CH₃)₃); 2,08 (td, J=14 et 6, 1H, H axial du -CH₂- en 5); 2,28 (dmt, 1H, H équatorial du -CH₂- en 5); 2,7 à 2,85 (mt, 4H, -CH₂- 1 et -CH₂- en 6); 3,27 (mt, 2H, -CH< en 3a et 1H du -CH₂- en 3); 3,9 à 4,05 (mt, 2H, -CH< en 7a et l'autre H du -CH₂- en 3); 7,1 à 7,7 (mt, 10H aromatiques).

### Exemple 4

A une suspension de 0,8 g de chlorhydrate de diphényl-7,7 (méthoxy-2 phényl)-4 perhydroisoindolol-4-(3aS,4S,7aS) dans 80 cm³ de dichlorométhane sec on ajoute 0,025 g d'hydroxy-1 benzotriazole, 0,27 cm³ d'acide phényl-2 propanoïque-(S), 0,32 cm³ de diisopropyléthylamine, puis on refroidit cette solution à +5°C et on ajoute rapidement une suspension de 0,43 g de chlorhydrate de (diméthylamino-3 propyl)1 éthyl-3 carbodiimide dans 10 cm³ de dichlorométhane sec. Le mélange réactionnel est agité 2 heures à +5°C, 18 heures à température ambiante, lavé par 20 cm³ d'eau, puis lavé par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 2,8 cm, hauteur 20 cm), en éluant sous une pression de 0,5 bar d'azote par un mélange de cyclohexane et d'acétate d'éthyle (65/35 en volumes) et en recueillant des fractions de 25 cm^{3.} Les fractions 6 à 12 sont réunies puis concentrés à sec sous pression réduite (2,7 kPa). Le rédidu est rituré dans de l'oxyde de diisopopyle. On obtient 0,53 g de diphényl-7,7 (méthoxy-2 phényl)-4 [phényl-2 propionyl-(S)]-2 perhydroisoindole4(3aS,4S,7aS) sous la forme de cristaux blancs fondant avec décomposition à 128°C.

### Exemple 5

A une suspension de 0,75 g de chlorhydrate de diphényl-7,7 (méthoxy-2 phényl)-4 perhydroisoindolol-4-(3aS,4S,7aS) dans 60 cm³ de dichlorométhane sec on ajoute 0,024 g d'hydroxy-1 benzotriazole, 0,33 g d'acide acétyloxy-2 phényl-2 acétique-(S), puis on refroidit cette solution à +5°C et on ajoute rapidement une suspension de 0,4 g de chlorhydrate de (diméthylamino-3 propyl)-1 éthyl-3 carbodiimide dans 20 cm³ de dichlorométhane sec, puis une solution de 0,63 cm³ de diisopropyléthylamine dans 20 cm³ de dichlorométhane sec. Le mélange réactionnel est agité 2 heures à +5°C, 24 heures à température ambiante, dilué par 120 cm³ de dichlorométhane, lavé par 100 cm³ d'eau, puis par 100 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm, diamètre 2,5 cm, hauteur 39 cm), en éluant sous une pression de 0,5 bar d'azote par un mélange de cyclohexane et d'acétate d'éthyle (50/50 en volumes) et en recueillant des fractions de 60 cm³. Les fractions 6 à 10 sont réunies puis concentrées à sec sous pression réduite (2,7 kPa). On obtient 0,96 g de diphényl-7,7 (méthoxy-2 phényl)-4 [acétyloxy-2 phényl-2 acétyl-(S)]-2 perhydroisoindolol-4-(3aS,4S,7aS) sous la forme d'une meringue blanche.

Spectre RMN du proton (DMSO d₆): A température ambiante, on observe le mélange des deux rotamères): 1,25 (dmt, J=14, H équatorial du-CH₂- en 5); 1,4 (dmt, J=14, H équatorial du -CH₂- en 5); 2,01 (s, 3H, -OCOCH₃); 2,27 (mt, 2H, H du -CH₂- en 5 et 1H du -CH₂- en 6); 2,65 à 3,6 (mt,-CH₂- et -CH<); 3,22 (s, 3H,-OCH₃); 4,38 (s, OH d'un rotamère); 4,86 (s, OH de l'autre rotamère); 5,66 (s, -CO-CH-O d'un rotamère); 5,88 (s, -CO-CH-O de l'autre rotamère); 6,6 à 7,6 (mt, 19H aromatiques).

Spectre infra-rouge (KBr), bandes caractéristiques (cm⁻¹) : 3400, 3100-3000, 3000-2850, 2830, 1740, 1660, 1600, 1580, 1495, 1450, 1235, 1050, 755, 700.

A une solution de 0,8 g de diphényl-7,7 (méthoxy-2 phényl)-4 [acétyloxy-2 phényl-2 acétyl-(S)]-2 perhydroisoindolol-4-(3aS,4S,7aS) dans 30 cm³ d'éthanol on ajoute 1,4 cm³ d'une solution aqueuse de soude 1N, puis 10 cm³ d'eau. Le mélange réactionnel est porté au reflux pendant 1 heure, concentré à sec sous pression réduite (2,7 kPa), repris par 50 cm³ d'eau, puis 1,5 cm³ de solution aqueuse d'acide chlorhydrique 1N, et extrait par 40 cm³ d'acétate d'éthyle (3 fois). Les phases organiques sont réunies, lavées par 50 cm³ d'eau, séchées sur sulfate de magnésium et concentrées à sec sous pression réduite (2,7 kPa). Le résidu est chromatographié sur colonne de gel de silice Merck (granulométrie 0,04-0,06 mm, diamètre 2,5 cm, hauteur 27 cm), en éluant sous une pression de 0,4 bar d'azote par un mélange de cyclohexane et d'acétate d'éthyle (50/50 en volumes) et en recueillant des fractions de 30 cm³. Les fractions 2 à 5 sont réunies et concentrées à sec sous pression réduite (2,7 kPa). Le résidu est trituré dans de l'oxyde d'isopropyle. On obtient 0,6 g de diphényl7,7 (méthoxy-2 phényl)-4 [hydroxy-2 phényl-2 acétyl-(S)]-2 perhydroisoindolol-4-(3aS,4S,7aS) sous la forme de cristaux blancs fondant à 256°C.

### Exemple 6

En opérant selon l'exemple 4, à partir de 2,25 g de chlorhydrate de diphényl-7,7 (méthoxy-2 phényl)-4 perhydroisoindolol-4-(3aS,4S,7aS) et de 1,25 g d'acide tert-butoxycarbonylamino-2 phényl-2 acétique-(S) on obtient 0,35 g de diphényl-7,7 (méthoxy-2 phényl)-4 [tertbutoxycarbonylamino-2 phényl-2 acétyl-(S)]-2 perhydroisoindolol-4(3aS,4S,7aS) sous la forme d'une meringue blanche.

Spectre RMN du proton (DMSO d₆): A température ambiante, on observe le mélange des rotamères: 1,3 et 1,4 (2s, -C(CH₃)₃); 1,3 à 1,7 (mt, 1H du -CH₂- en 5); 2,15 à 2,45 (mt, 2H, l'autre H du -CH₂- en 5 et un H du -CH₂-en 6); 2,7 à 3,7 (mt, -CH₂- et -CH<); 3,33 et 3,4 (2s,-OCH₃); 4,84 et 5,11 (2s, 1H du NCO-CH-N des deux isomères); 6,6 à 7,7 (mt, 19H aromatiques).

Spectre infra-rouge (KBr), bandes caractéristiques (cm⁻¹) : 3580, 3550-3450, 3420, 3100-3000, 3000-2850, 2835, 1710, 1645, 1600, 1580, 1490, 1455, 1395, 1370, 1240, 1170, 1030.

En opérant selon l'exemple 1 à partir de 0,62 g de diphényl-7,7 (méthoxy-2 phényl)-4 [tert-butoxycarbonylamino-2 phényl-2 acétyl(S)]-2 perhydroisoindolol-4-(3aS,4S,7aS) on obtient 0,54 g de chlorhydrate de diphényl-7,7 (méthoxy-2 phényl)-4 [amino-2 phényl-2 acétyl-(S)]-2 perhydroisoindolol-4-(3aS,4S,7aS) sous la forme d'une meringue blanche.

Spectre RMN du proton (DMSO d₆): On observe le mélange de diastéréoisomères: 1,34 et 1,53 (2dmt, J=14, 1H en totalité, H équatorial du -CH₂- en 5 pour les deux isomères); 2,31 (mt, 2H, l'autre H du -CH₂- en 5 et 1H du -CH₂- en 6); 2,8 à 3,7 (mt, -CH₂- et -CH<); 3,36 et 3,42 (2s, 3H, -OCH₃); 4,76 et 4,95 (2s, 1H, NCO-CH-N des deux isomères); 6,6 à 7,7 (mt, 19H aromatiques).

Spectre infra-rouge (KBr), bandes caractéristiques (cm⁻¹) : 3425, 3100-3000, 3000-2850, 2830, 3150-2500, 1660, 1600, 1585, 1495, 1450, 1235, 1030, 755, 700.

### Exemple 7

A une solution de 0,28 g d'acide (méthoxy-2 phényl)-acétique dans 20 cm³ de dichlorométhane sec on ajoute 0,28 g de carbonyldiimidazole. On agite à température ambiante pendant 1 heure, puis on ajoute successivement une suspension de 0,7 g de chlorhydrate de triphényl4,7,7 perhydroisoindolol-4-(3aS,4S,7aS) dans 20 cm³ de dichlorométhane sec, puis 0,48 cm³ de triéthylamine. Le mélange réactionnel est agité 24 heures à température ambiante, dilué par 100 cm³ de dichlorométhane, lavé par 100 cm³ d'eau (deux fois), puis par 100 cm³ d'une solution aqueuse saturée en chlorure de sodium, séché sur sulfate de magnésium, et concentré à sec sous pression réduite (2,7 kPa). Le résidu est chromatographié sur colonne de gel de silice Merck (granulométrie 0,04-0,06 mm, diamètre 2 cm, hauteur 16 cm), en éluant sous une pression de 0,5 bar d'azote par un mélange de cyclohexane et d'acétate d'éthyle (55/45 en volumes) et en recueillant des fractions de 20 cm³. Les fractions 7 à 8 sont réunies puis concentrées à sec sous pression réduite (2,7 kPa). Le résidu est cristallisé dans 16 cm³ d'acétonitrile. Les cristaux sont essorés et séchés. On obtient 0,3 g de triphényl-4,7,7 (méthoxy-2 phényl)-acétyl-2 perhydroisoindolol-4-(3aS,4S,7aS) sous la forme de cristaux blancs fondant à 236°C.

Le chlorhydrate de triphényl-4,7,7 perhydroisoindolol-4-(3aS,4S,7aS) peut ête préparé de la manière suivante :

A une solution de 6,8 g de triphényl-4,7,7 tert-butoxycarbonyl-2 perhydroisoindolol-4-(3aS,4S,7aS) dans 60 cm³ de dioxanne on ajoute à température ambiante une solution de 115 cm³ de dioxanne chlorhydrique 6,3 N. Le mélange réactionnel est agité 2 heure à cette température, puis concentré à sec sous pression réduite (2,7 kPa). Le résidu est lavé par de l'acétonitrile, essoré puis séché. On obtient 4 g de chlorhydrate de triphényl-4,7,7 perhydroisoindolol-4(3aS,4S,7aS) sous la forme d'une meringue blanche.

Spectre RMN du proton (DMSO d₆) : 1,51 (td, J=14, 1H, H axial du -CH₂- en 5); 1,72 (d large, J=14, 1H, H équatorial du -CH₂- en 5); 2,34 (d large, J=14, 1H, H équatorial du -CH₂- en 6); 2,42 (td, J=10, 1H du _CH₂- en 1); 2,87 (td, J=14, 1H, H axial du -CH₂- en 6); 2,94 (mt, 1H, -CH< en 3a); 3,05 à 3,25 (mt, 3H, l'autre H du -CH₂- en 1 et le-CH₂- en 3); 3,57 (mt, 1H, -CH< en 7a); 5,67 (mf, 1H, OH); 7,1 à 7,6 (mt, 15H aromatiques); 8,9 (2mf, 1H chacun, NH₂⁺)

Spectre infra-rouge (KBr), bandes caractéristiques (cm ⁻¹) : 3500-3250, 3100-3000, 3000-2825, 2800-2250, 1600, 1580, 1495, 1445, 1075, 750, 700.

Le triphényl-4,7,7 tert-butoxycarbonyl-2 perhydroisoindo-lol-4-(3aS,4S,7aS) peut être préparé de la manière suivante :

A une solution de 11,66 g de diphényl-7,7 tert-butoxycarbonyl-2 perhydroisoindolone-4-(3aS,7aS) dans 70 cm³ de tétrahydrofuranne sec, on ajoute goutte à goutte à température ambiante sous agitation une suspension de 10,78 g de bromure de phénylmagnésium dans 65 cm³ d'oxyde de diéthyle. Le mélange réactionnel est agité à température ambiante pendant 24 heures, porté au reflux pendant 5 heures, puis traité par 250 cm³ d'une solution aqueuse saturée en chlorure d'ammonium, dilué par 200 cm³ d'acétate d'éthyle, lavé par 200 cm³ d'eau (deux fois), puis par 200 cm³ d' une solution aqueuse saturée en chlorure de sodium, séché sur sulfate de magnésium, filtré et concentré à sec sous pression réduite (2,7 kPa). Le résidu est chromatographié sur colonne de gel de silice Merck (granulométrie 0,04-0,06 mm, diamètre 5,3 cm, hauteur 31,5 cm), en éluant sous une pression de 0,5 bar d'azote par un mélange de cyclohexane et d'acétate d'éthyle (90/10 en volumes) et en recueillant des fractions de 100 cm³. Les fractions 23 à 48 sont réunies et concentrées à sec sous pression réduite (2,7 kPa). On obtient 5,75 g de triphényl-4,7,7 tertbutoxycarbonyl-2 perhydroisoindolol-4- (3aS,4S,7aS) sous forme d'une meringue jaune pâle.

Spectre RMN du proton (DMSO d₆): 1,37 (s, 9H, -C(CH₃)₃); 1,65 (mt, 2H, -CH₂- en 5); 2,28 (d large, J=14, 1H, H équatorial du -CH₂- en 6); 2,65 (t, J=9, 1H du -CH₂- en 1); 2,85 (mt, 1H, -CH< en 3a); 3,05 (td, J=14 et 3,5; 1H, H axial du -CH₂- en 6); 3,25 (mt, 2H, l'autre H du -CH₂- en 1 et 1H du -CH₂- en 3); 3,4 (d, J=11, 1H, l'autre Hdu-CH₂- en 3); 3,5 (mt, 1H, -CH< en 7a); 4,4 (s, 1H, OH); 7,1 à 7,6 (mt, 15H aromatiques).

Spectre infra-rouge (KBr), bandes caractéristiques (cm⁻¹) : 3425, 3100-3000, 3000-2850, 1680, 1600, 1580, 1495, 1475, 1410, 1365, 1170, 750, 700.

### Exemple 8

En opérant selon l'exemple 4, à partir de 1,13 g de chlorhydrate de diphényl-7,7 (méthoxy-2 phényl)-4 perhydroisoindolol-4-(3aS,4S,7aS) et de 0,42 g d'acide (méthoxy-2 phényl)-acétique on obtient 0,61 g de diphényl-7,7 (méthoxy-2 phényl)-4 [(méthoxy-2 phényl)-acétyl]-2 perhydroisoindolol-4-(3aS,4S,7aS) sous la forme de cristaux blancs fondant à 204°C.

### Exemple 9

En opérant selon l'exemple 4, à partir de 0,75 g de chlorhydrate de diphényl-7,7 (méthoxy-2 phényl)-4 perhydroisoindolol-4-(3aS,4S,7aS) et de 0,28 g d'acide (méthoxy-3 phényl)-acétique on obtient 0,54 g de diphényl-7,7 (méthoxy-2 phényl)-4 [(méthoxy-3 phényl)-acétyl]-2 perhydroisoindolol-4-(3aS,4S,7aS) sous la forme de cristaux blancs fondant à 185°C.

### Exemple 10

En opérant selon l'exemple 4, à partir de 0,75 g de chlorhydrate de diphényl-7,7 (méthoxy-2 phényl)-4 perhydroisoindolol-4-(3aS,4S,7aS) et de 0,28 g d'acide (méthoxy-4 phényl)-acétique on obtient 0,61 g de diphényl-7,7 (méthoxy-2 phényl)-4 [(méthoxy-4 phényl)-acétyl]-2 perhydroisoindolol-4-(3aS,4S,7aS) sous la forme de cristaux blancs fondant à 211°C.

### Exemple 11

En opérant selon l'exemple 4, à partir de 0,8 g de chlorhydrate de diphényl-7,7 (méthoxy-2 phényl)-4 perhydroisoindolol-4-(3aS,4S,7aS) et de 0,38 g d'acide naphtyl-1 acétique on obtient 1,16 g de diphényl-7,7 (méthoxy-2 phényl)-4 (naphtyl-1 acétyl)-2 perhydroisoindolol4-(3aS,4S,7aS) sous la forme d'un solide blanc fondant à 225°C.

### Exemple 12

En opérant selon l'exemple 4, à partir de 0,8 g de chlorhydrate de diphényl-7,7 (méthoxy-2 phényl)-4 perhydroisoindolol-4-(3aS,4S,7aS) et de 0,34 g d'acide thiényl-3 acétique on obtient 0,53 g de diphényl-7,7 (méthoxy-2 phényl)-4 [(thiényl-3)-acétyl]-2 perhydroisoindolol-4-(3aS,4S,7aS) sous la forme de cristaux blancs fondant avec décomposition à 106°C.

### Exemple 13

A une solution de 0,44 g d'acide indolyl-3 acétique dans 20 cm³ de dichlorométhane sec on ajoute 0,41 g de cabonyldiimidazole. On agite à température ambiante pendant 1 heure, puis on ajoute successivement une suspension de 1,1 g de chlorhydrate de diphényl-7,7 (méthoxy-2 phényl)-4 perhydroisoindolol-4-(3aRS,4RS,7aRS) dans 25 cm³ de dichlorométhane sec, puis 0,7 cm³ de triéthylamine. Le mélange réactionnel est agité 24 heures à température ambiante, dilué par 100 cm³ de dichlorométhane, lavé par 100 cm³ d'eau (deux fois), puis par 100 cm³ d'une solution aqueuse saturée en chlorure de sodium, séché sur sulfate de magnésium, et concentré à sec sous pression réduite (2,7 kPa). Le résidu est chromatographié sur colonne de gel de silice Merck (granulométrie 0,04-0,06 mm, diamètre 2 cm, hauteur 22 cm), en éluant sous une pression de 0,5 bar d'azote par un mélange de cyclohexane et d'acétate d'éthyle (35/65 en volumes) et en recueillant des fractions de 20 cm³. Les fractions 7 à 8 sont réunies puis concentrées à sec sous pression réduite (2,7 kPa). Le résidu est trituré dans de l'oxyde de diisopropyle. On obtient 0,17 g de diphényl-7,7 (indolyl-3 acétyl)-2 (méthoxy-2 phényl)-4 perhydroisoindolol-4(3aRS,4RS,7aRS) sous la forme d'un solide blanc.

Spectre RMN du proton (DMSO d₆): 1,48 (d large, J=14,5,1H, H équatorial du -CH₂- en 5); 2,27 (d large, J=14,5, 1H, H équatorial du -CH₂- en 6); 2,32 (td, J=14,5 et 2, 1H, H axial du -CH₂- en 5); 3,02 (td, J=14,5 et 2, 1H, H axial du -CH₂- en 6); 2,88 et 3,2 à 3,7 (2mt, respectivement 1H et 5H, -CH₂- et -CH); 3,44 (s, 3H, -OCH₃); 3,52 (s, 2H, -N-COCH₂-); 6,8 à 7,6 (mt, 19H, aromatiques).

Spectre infra-rouge (KBr), bandes caractéristiques (cm⁻¹) : 3425, 3125-3000, 3000-2850, 1625, 1585, 1490, 1460, 1235, 1030, 745, 700

### Exemple 14

A une suspension de 0,96 g de chlorhydrate de diphényl-7,7 (méthoxy-2 phényl)-4 perhydroisoindolol-4-(3aS,4S,7aS) dans 25 cm³ de dichlorométhane sec, on ajoute successivement à température ambiante sous agitation une solution de 0,43 g de chlorure d'(indolyl-3)-2 oxo-2 acétyle dans 20 cm³ de dichlorométhane sec, puis une solution de 0,6 cm³ de triéthylamine dans 5 cm³ de dichlorométhane sec. Le mélange réactionnel est agité 24 heures à cette température, puis dilué par 200 cm³ de dichlorométhane, lavé par 100 cm³ de solution aqueuse de soude 1N, par 50 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm, diamètre 2,5 cm, hauteur 31 cm), en éluant sous une pression de 0,4 bar d'azote par un mélange de dichloro-1,2 éthane et de méthanol (70/30 en volumes) et en recueillant des fractions de 15 cm³. Les fractions 2 à 9 sont réunies et concentrées à sec sous pression réduite (2,7 kPa). On obtient 1,15 g de diphényl-7,7 [oxo-2 (indolyl-3)-2 acétyl]-2 (méthoxy2 phényl)-4 perhydroisoindolol-4-(3aS,4S,7aS) sous la forme d'une meringue orange.

Spectre infra-rouge (KBr), bandes caractéristiques (cm ⁻¹) : 3400, 3250, 3100-3000, 3000-2850, 2835, 1650-1600, 1580, 1520, 1490, 1455, 1235, 1030, 755, 700.

A une solution de 1,1 g de diphényl-7,7 [oxo-2 (indolyl-3)-2 acétyl(RS)]-2 (méthoxy-2 phényl)-4 perhydroisoindolol-4-(3aS,4S,7aS) dans 35 cm³ d'éthanol on ajoute, à température ambiante sous agitation, 0,38 g de borohydrure de sodium. Le mélange réactionnel est agité 4 heures à cette température, puis traité par 2 cm³ d'acide acétique et concentré à sec sous pression réduite (2,7 kPa). Le résidu est dissous dans 100 cm³ d'acétate d'éthyle, la phase organique est lavée par 50 cm³ d'une solution aqueuse de soude 0,1N, par 50 cm³ d'eau, puis par 50 cm³ d'une solution aqueuse saturée en chlorure de sodium, séchée sur sulfate de magnésium et concentrée à sec sous pression réduite (2,7 kPa). Le résidu est chromatographié sur colonne de gel de silice Merck (granulométrie 0,04-0,06 mm, diamètre 2,8 cm, hauteur 27 cm), en éluant sous une pression de 0,4 bar d'azote par un mélange de dichloro-1,2 éthane et de méthanol (96/4 en volumes) et en recueillant des fractions de 10 cm³. Les fractions 17 à 31 sont réunies et concentrées à sec sous pression réduite (2,7 kPa). Le résidu est trituré dans de l'oxyde de diisopropyle. On obtient 0,45 g de diphényl7,7 [hydroxy-2 (indolyl-3)-2 acétyl-(RS)]-2 (méthoxy-2 phényl)-4 perhydroisoindolol-4-(3aS,4S,7aS) sous la forme d'une meringue blanche.

Spectre RMN du proton (DMSO d₆): A température ambiante, on observe un mélange d'isomères et de rotamères: 1,4 (mt, 1H, H équatorial du-CH2- en 5); 2,3 (mt, 2H, H axial du -CH₂- en 5 et 1H du -CH2- en 6); 2,5 à 3,8 (mt, -CH₂- et -CH<); 3,30-3,32-3,35 et 3,38 (4s, OCH₃ des différents isomères et rotamères); 5-5,12-5,24 et 5,28 (4s, 1H, >N_Co-CH-O des différents isomères et rotamères); 6,5 à 7,8 (mt aromatiques).

Spectre infra-rouge (KBr), bandes caractéristiques (cm ⁻¹) : 3420, 3125-3000, 3000-2850, 2830, 1630, 1600, 1580, 1495, 1450, 1235, 1030, 755, 745, 700.

Exemple 15 En opérant selon le mode opératoire de l'exemple 4, à partir de 0,8 g de chlorhydrate de diphényl-7,7 (méthoxy-2 phényl)-4 perhydroisoindolol-4-(3aS,4S,7aS) et de 0,39 g d'acide (fluoro-5 indolyl-3)-acétique on obtient 0,36 g de diphényl-7,7 [(fluoro-5 indolyl-3)-acétyl]-2 (méthoxy-2 phényl)-4 perhydroisoindo-lol-4(3aS,4S,7aS) sous la forme de cristaux blancs fondant avec décomposition à 170°C.

### Exemple 16

En opérant selon l'exemple 4, à partir de 0,8 g de chlorhydrate de diphényl-7,7 (méthoxy-2 phényl)-4 perhydroisoindolol-4-(3aS,4S,7aS) et de 0,41 g d'acide (méthoxy-5 indolyl-3)-acétique on obtient 0,66 g de diphényl-7,7 [(méthoxy-5 indolyl-3)- acétyl]-2 (méthoxy-2 phényl)4 perhydroisoindolol-4-(3aS,4S,7aS) sous la forme d'une meringue beige.

Spectre RMN du proton (DMSO d₆) : 1,5 (d large, J=14, 1H, H équatorial du -CH₂- en 5); 2,29 (d large, J=14, 1H, H équatorial du -CH₂- en 6); 2,35 (td, J=14 et 2,5; 1H axial du -CH₂- en 5); 3,04 (td, J=14 et 2,5; H axial du -CH₂- en 6); 2,8 à 3,9 (mt, -CH₂- et -CH<); 3,44 (s, -OCH₃); 3,75 (s, NCO-CH₂-); 3,89 (s, 3H, -OCH₃ de l'indole); 6,7 à 7,7 (mt, 18H aromatiques); 10,3 (mf, 1H, NH de l'indole).

Spectre infra-rouge (KBr), bandes caractéristiques (cm⁻¹): 3300-2200, 3125-3000, 3000-2850, 2830, 1625, 1600, 1580, 1485, 1450, 1230, 1215, 1025, 755, 700.

### Exemple 17

En opérant selon l'exemple 4, à partir de 0,75 g de chlorhydrate de diphényl-7,7 (méthoxy-2 phényl)-4 perhydroisoindolol-4-(3aS,4S,7aS) et de 0,32 g d'acide (méthyl-1 indolyl-3)-acétique on obtient 0,56 g de diphényl-7,7 (méthoxy-2 phényl)-4 [(méthyl-1 indolyl-3)-acétyl)-2 perhydroisoindolol-4-(3aS,4S,7aS) sous la forme d'une meringue beige.

Spectre RMN du proton (DMSO d₆): A température ambiante, on observe le mélange des deux rotamères): 1,42 (mt, 1H, 1H du -CH₂- en 5); 2,31 (mt, 2H, l'autre H du -CH₂- en 5 et 1H du -CH₂- en 6); 2,94 (mt, l'autre H du -CH₂- en 6); 2,7 à 3,6 (mt, -CH₂- et -CH<); 3,37 (s, 3H, -OCH₃); 3,45 et 3,5 (2s, 2H, -COCH₂Ar); 3,72 et 3,78 (2s, 3H, NCH₃); 6,8 à 7,7 (mt, 19H aromatiques).

Spectre infra-rouge (KBr), bandes caractéristiques (cm⁻¹) : 3400, 3125-3000, 3000-2850, 2830, 1637, 1600, 1580, 1485, 1450, 1235, 1050, 750, 700.

### Exemple 18

A une solution de 4,5 g de chlorhydrate de diphényl-7,7 (méthoxy-2 phényl)-4 perhydroisoindolediol-4,5-(3aRS,4RS,5RS,7aRS) dans 100 cm³ de dichlorométhane, refroidie à 0°C, on ajoute 4,2 cm³ de triéthylamine puis 2,4 g du chlorure de l'acide (méthoxy-2 phényl)acétique dans 50 cm³ de dichlorométhane. On agite à température ambiante pendant 90 minutes; le mélange réactionnel est lavé par 2 fois 10 cm³ d'eau, séché sur sulfate de magnésium et concentré à sec sous pression réduite (2,7 kPa). Le solide cristallisé est repris par 100 cm³ d'oxyde de diisopropyle puis filtré, lavé par 50 cm³ d'une solution saturée de bicarbonate de sodium puis 50 cm³ d'oxyde de diisopropyle. On obtient 4,35 g de diphényl-7,7 (méthoxy-2 phényl)-4 (méthoxy-2 phényl) acétyl-2 perhydroisoindolediol-4,5-(3aRS,4RS,5RS,7aRS) sous forme d'un solide beige clair fondant à 278°C.

Le chlorure de l'acide (méthoxy-2 phényl)acétique est obtenu à partir d'un mélange de 2,2 g d'acide (méthoxy-2 phényl)acétique et de 20 cm³ de chlorure de thionyle, porté à reflux 30 minutes. Après concentration à sec sous pression réduite (2,7 kPa), on obtient 2,4 g d'une huile jaune utilisée à l'état brut dans les synthèses ultérieures.

Le chlorhydrate de diphényl-7,7 (méthoxy-2 phényl)-4 perhydroisoindolediol-4,5 (3aRS,4RS,5RS,7aRS) peut être préparé de la manière suivante :

A une solution de 5,15 g de diphényl-7,7 (méthoxy-2 phényl)-4 tertbutoxycarbonyl-2 perhydroisoindolediol-4,5-(3aRS,4RS,5RS,7aRS) dans 25 cm³ de dioxanne, on ajoute à température ambiante une solution de 25 cm³ de dioxanne chlorhydrique 6N. Le mélange réactionnel est agité 1 heure à cette température, puis concentré à sec sous pression réduite (2,7 kPa). Le résidu est lavé par 20 cm³ l'acétonitrile, essoré et séché. On obtient 4,5 g de chlorhydrate de diphényl-7,7 (méthoxy-2 phényl)-4 perhydroisoindolediol-4,5-(3aRS,4RS,5RS,7aRS) sous forme de cristaux blancs fondant à une température supérieure à 300°C.

Le diphényl-7,7 (méthoxy-2 phényl)-4 tert-butoxycarbonyl-2 perhydroisoindolediol-4,5-(3aRS,4RS,5RS,7aRS) peut être préparé de la manière suivante :

A une suspension de 26,4 g d'acétoxy-5 diphényl-7,7 (méthoxy-2 phényl)-4 tert-butoxycarbonyl-2 perhydroisoindolone-4-(3aRS,5RS,7aRs) et de 43,3 g de chlorure de cérium anhydre dans 265 cm³ de tétrahydrofuranne sec, on ajoute goutte à goutte à température ambiante sous agitation une suspension de 30,9 g de bromure de méthoxy-2 phénylmagnésium dans 170 cm³ de tétrahydrofuranne sec. Le mélange réactionnel est agité à température ambiante pendant 24 heures, traitée par 400 cm³ d'une solution aqueuse saturée en chlorure d'ammonium, repris par 1000 cm³ d'acétate d'éthyle puis filtré sur célite. La phase organique est décantée, séchée sur sulfate de magnésium, filtrée et concentrée à sec sous pression réduite (2,7 kPa). Le résidu est chromatographié sur colonne de gel de silice Merck (granulométrie 0,04-0,06 mm, diamètre 7 cm, hauteur 55 cm), en éluant sous une pression de 0,5 bar d'azote par un mélange de cyclohexane et d'acétate d'éthyle (70/10 en volumes) et en recueillant des fractions de 250 cm³. Les fractions 10 à 19 sont réunies et concentrées à sec sous pression réduite (2,7 kPa). On obtient 18 g de diphényl-7,7 (méthoxy-2 phényl)-4 tert-butoxycarbonyl-2 perhydroisoindolediol-4,5-(3aRS,4RS,5RS,7aRS) sous forme de cristaux blancs fondant à 229°C.

L'acétoxy-5 diphényl-7,7 tert-butoxycarbonyl-2 perhydroisoindolone-4-(3aRS,5RS,7aRS) peut être préparé de la manière suivante :

A une suspension de 19 g de chlorhydrate d'acétoxy-5 diphényl-7,7 perhydroisoindolone-4-(3aRS,5RS,7aRS) dans 200 cm³ de dichlorométhane sec, on ajoute à une température voisine de 5°C, sous agitation, 46,9 cm³ de triéthylamine, 11,8 g de di-tert-butyl dicarbonate, puis 0,3 g de diméthylamino-4 pyridine. Le mélange réactionnel est agité à température ambiante pendant 24 heures puis lavé par une solution aqueuse saturée en bicarbonate de sodium. La phase organique est décantée, séchée sur sulfate de magnésium, filtrée et concentrée à sec sous pression réduite (2,7 kPa). Le résidu est cristallisé dans 120 cm³ d'oxyde de diisopropyle. On obtient 21 g d'acétoxy-5 diphényl-7,7 perhydroisoindolone-4-(3aRs,5Rs,7aRS) sous forme de cristaux blancs fondant à 213°C.

Le chlorhydrate d'acétoxy-5 diphényl-7,7 perhydroisoindo-lone-4-(3aRS,5RS,7aRS) peut être préparé de la manière suivante :

A une solution de 51,2 g d'acétoxy-5 diphényl-7,7 vinyloxycarbonyl-2 perhydroisoindolinone-4-(3aRS,5RS 7aRS) dans 118 cm³ de dioxanne, on ajoute à température ambiante, une solution de 394 cm³ de dioxanne chlorhydrique 5,2 N. Le mélange réactionnel est agité 1 heure à cette température, puis concentré à sec sous pression réduite (2,7 kPà). Le résidu est recristallisé dans 200 cm³ d'éthanol bouillant. On obtient 13,4 g de chlorhydrate d'acétoxy-5 diphényl-7,7 perhydroisoindolone4-(3aRS,5RS,7aRS) sous forme de cristaux blancs fondant à une température supérieure à 300°C.

L'acétoxy-5 diphényl-7,7 vinyloxycarbonyl-2 perhydroisoindolino-ne-4-(3aRS,5RS,7aRS) peut être préparé de la manière suivante :

A une solution de 58 g d'acétoxy-5 benzyl-2 diphényl-7,7 perhydroisoindolinone-4-(3aRS,5RS,7aRS) dans 580 cm³ de dichlorométhane sec, on ajoute à température ambiante sous agitation 13,6 cm³ de chloroformiate de vinyle. Le mélange réactionnel est porté à reflux du solvant pendant une heure, refroidi à température ambiante et concentré à sec sous pression réduite (2,7 kPa). Le résidu est cristallisé dans 400 cm³ d'un mélange d'oxyde de diisopropyle et d'éther de pétrole (50/50 en volumes). On obtient 51,4 g d'acétoxy-5 diphényl-7,7 vinyloxycarbonyl-2 perhydroisoindolone-4-(3aRs,5Rs,7aRs) sous forme de cristaux jaunes fondant à 205-210°C.

L'acétoxy-5 benzyl-2 diphényl-7,7 perhydroisoindolone-4-(3aRS,5RS,7aRS) peut être préparé de la manière suivante :

A une solution de 86 g d'acétoxy-6 diphényl-4,4 cyclohexènone-2 et de 96 cm³ de N-butoxyméthyl N-triméthylsilylméthyl benzylamine dans 1000 cm³ de dichlorométhane, on ajoute 15 gouttes d'acide trifluoroacétique. Le mélange réactionnel est agité à température ambiante 15 heures puis on ajoute 2 g de carbonate de sodium et concentre à sec sous pression réduite (2,7 kPa). Le résidu est chromatographié sur colonne de gel de silice Merck (granulométrie 0,04-0,06 mm, diamétre 7 cm, hauteur 70 cm), en éluant sous une pression de 0,5 bar d'azote par un mélange de cyclohexane et d'acétate d'éthyle (20/80 en volumes) et en recueillant des fractions de 200 cm³. Les fractions 6 à 10 sont réunies et concentrées à sec sous pression réduite (2,7 kPa). On obtient 70 g d'acétoxy-5 benzyl-2 diphényl-7,7 perhydroisoindolone-4-(3aRs,5Rs,7aRS) sous forme de miel (point de fusion inférieur à 40°C).

La N-butoxyméthyl N-triméthylsilylméthyl benzylamine peut être préparée selon la méthode de Y. TERAO et coll., Chem. Pharm. Bull., 33, 2762 (1985).

L'acétoxy-6 diphényl-4,4 cyclohexènone-2 peut être préparée selon la méthode décrite par W.OPPOLZER et coll., Helv. Chim. Acta, 59, 2012 (1976).

### Exemple 19

En opérant selon l'exemple 18 à partir de 0,5 g chlorhydrate de diphényl-7,7 phényl-4 perhydroisoindolediol-4,5-(3aS,4S,5S,7aS) et de 0,39 g d'acide (méthoxy-2 phényl)-acétique, on obtient 0,4 g de diphényl-7,7 phényl-4 (méthoxy-2 phényl) acétyl-2 perhydroisoindolediol-4,5-(3aS,4S,5S,7aS) sous forme d'un solide blanc fondant avec décomposition à 150°C.

### Exemple 20

En opérant selon l'exemple 1 à partir de 0,5 g chlorhydrate de diphényl-7,7 phényl-4 perhydroisoindolediol-4,5-(3aS,4S,5S,7aS) et de 0,25 g d'acide (méthoxy-2 phényl)-2 propionique-(S), on obtient 0,52 g de diphényl-7,7 phényl-4 [(méthoxy-2 phényl)-2 propionyl- (S)]-2 perhydroisoindolediol-4,5-(3aS,4S,5S,7aS) sous forme d'un solide blanc fondant avec décomposition à 158°C.

### Exemple 21

A une solution de 0,9 g de chlorhydrate de diphényl-7,7 (méthoxy-2 phényl)-4 perhydroisoindolediol-4,5-(3aRS,4RS,5RS,7aRS), de 0,4 g d'acide indolyl-3 acétique et de 20 mg d'hydrate d' hydroxy-1benzotriazole dans 90 cm³ de dichlorométhane, refroidie à 0°C, on ajoute 0,46 g de (diméthylamino-3 propyl)-1 éthyl-3 carbodiimide et 0,34 cm³ de diisopropylamine. On agite à température ambiante pendant 15 heures, acidifie par HCl 0,1 N puis reprend par une solution aqueuse saturée de chlorure de sodium. La phase organique est décantée, séchée sur sulfate de magnésium et concentrée à sec sous pression réduite (2,7 kPa). La meringue obtenue est recristallisée dans 10 cm³ d'acétonitrile bouillant. On obtient 0,85 g de diphényl-7,7 (méthoxy-2 phényl)-4 (indolyl-3) acétyl-2 perhydroisoindolediol-4,5-(3aRS,4RS,5RS,7aRS) sous forme d'un solide blanc fondant à 266°C.

### Exemple 22

A une suspension de 0,14 g de chlorhydrate de diphényl-7,7 fluoro-4 (méthoxy-2 phényl)-4 perhydroisoindole-(3aS,4S,7aS) dans 7 cm³ de dichlorométhane sec on ajoute à température ambiante 0,1 cm³ de triéthylamine, puis 0,04 cm³ de chlorure de phénylacétyle. Le mélange réactionnel est agité 5 heures à cette température, dilué par 100 cm³ de dichlorométhane, lavé par 40 cm³ d'une solution saturée en hydrogénocarbonate de sodium, puis par 40 cm³ d'eau, séché sur sulfate de magnésium et concentré à sec sous pression réduite (2,7 kPa). Le résidu obtenu est trituré dans de l'oxyde de diisopropyle. On obtient 0,1 g de diphényl-7,7 fluoro-4 (méthoxy-2 phényl)-4 phénylacétyl-2 perhydroisoindole-(3aS,4S,7aS) sous la forme d'une meringue blanche.

Spectre RMN du proton (DMSO D₆): A température ambiante, on observe un mélange de rotamères: 1,62 (mt, 1H, 1H du -CH₂- en 5); 2 à 3,8 (mt, _CH₂ - et -CH<); 3,38 et 3,42 (2s, 3H, -OCH₃); 6,7 à 7,6 (mt, 19H, aromatiques)

Spectre infra-rouge (KBr), bandes caractéristiques (cm⁻¹): 3100-3000, 3000-2850, 2840, 1640, 1600, 1580, 1495, 1455, 1240, 1030, 755, 720, 700

Le chlorhydrate de diphényl-7,7 fluoro-4 (méthoxy-2 phényl)-4 perhydroisoindole-(3aS,4S,7aS) peut être obtenu de la manière suivante :

A une solution de 2,07 g de diphényl-7,7 fluoro-4 (méthoxy-2 phényl)-4 tert-butoxycarbonyl-2 perhydroisoindole-(3aS,4S,7aS) dans 20 cm³ de dioxanne, on ajoute à température ambiante une solution de 20 cm³ de dioxanne chlorhydrique 6,3 N. Le mélange réactionnel est agité à cette température pendant 3 heures, puis concentré à sec sous pression réduite (2,7 kPa). Le résidu est cristallisé dans 40 cm³ d'éthanol absolu, les cristaux sont essorés puis séchés. On obtient 1 g de chlorhydrate de diphényl-7,7 fluoro-4 (méthoxy-2 phényl)-4 perhydroisoindole-(3aS,4S,7aS) sous la forme de cristaux blancs fondant à 270°C.

Le diphényl-7,7 fluoro-4 (méthoxy-2 phényl)-4 tert-butoxycarbonyl-2 perhydroisoindole-(3aS,4S,7aS) peut être obtenu de la manière suivante :

A une solution refroidie à 0°C de 6,48 g de diphényl-7,7 (méthoxy-2 phényl)-4 tert-butoxycarbonyl-2 perhydroisoindolol-4-(3aS,4S,7aS) dans 70 cm³ de dichlorométhane sec, on ajoute goutte à goutte une solution de 4,3 cm³ trifluorure de diéthylaminosoufre dans 20 cm³ de dichlorométhane sec. Le mélange réactionnel est agité 3 heures à cette température, lavé par 100 cm³ d'une solution aqueuse saturée en hydrogénocarbonate de sodium, puis par 100 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm, diamètre 4,8 cm, hauteur 26 cm), en éluant sous une pression de 0,5 bar d'azote par un mélange de cyclohexane et d'acétate d'éthyle (95/5 en volumes) et en recueillant des fractions de 50 cm³. Les fractions 34 à 63 sont réunies et concentrées à sec sous pression réduite (2,7 kPa). On obtient 2,2 g de diphényl-7,7 fluoro-4 (méthoxy-2 phényl)-4 tert-butoxycarbonyl-2 perhydroisoindo-le-(3aS,4S,7aS) sous la forme d'une meringue blanche.

### Exemple 23

A une solution de 1,5 g de chlorhydrate de diphényl-4,4 (méthoxy-2 phényl)-7 hexahydro-2,3,3a,4,5,7a isoindole-(3aS,7aR) dans 20 cm³ de dichlorométhane, refroidie à 0°C, on ajoute 1,5 cm³ de triéthylamine puis 1,6 g du chlorure de l'acide (méthoxy-2 phényl)acétique. On agite à température ambiante pendant 15 heures; le mélange réactionnel est lavé par 2 fois 40 cm³ d'eau, séché sur sulfate de magnésium et concentré à sec sous pression réduite (2,7 kPa). Le résidu est chromatographié sur colonne de gel de silice Merck (granulométrie 0,04-0,06 mm, diamètre 3,6 cm, hauteur 25 cm), en éluant sous une pression de 0,5 bar d'azote par un mélange de cyclohexane et d'acétate d'éthyle (75/25 en volumes) et en recueillant des fractions de 25 cm³. Les fractions 14 à 28 sont réunies et concentrées à sec sous pression réduite (2,7 kPa). La meringue jaune obtenue est recristallisée dans 10 cm³ de cyclohexane pour fournir 0,38 g de diphényl-4,4 (méthoxy-2 phényl)acétyl-2 (méthoxy-2 phényl)-7 hexahydro-2,3,3a,4,5,7a isoindole-(3aS,7aR) sous forme d'un solide beige fondant à 142°C.

Le chlorhydrate de diphényl-4,4 (méthoxy-2 phényl)-7 hexahydro2,3,3a,4,5,7a isoindole-(3aS,7aR) peut être obtenu de la manière suivante :

Une solution de 8,56 g de diphényl-7,7 (méthoxy-2 phényl)-4 tertbutoxycarbonyl-2 perhydroisoindolol-4-(3aS,4S,7aS) dans 53 cm³ d'acide acétique et 30 cm³ d'acide chlorhydrique 12 N est chauffée à 95°C pendant 45 minutes puis refroidie à température ambiante et concentrée à sec sous pression réduite (2,7 kPa). Le résidu est recristallisé dans 20 cm³ d'acétonitrile. On obtient 5,2 g de chlorhydrate de diphényl-4,4 (méthoxy-2 phényl)-7 hexahydro-2,3,3a,4,5,7a isoindole-(3aS,7aR) sous forme de cristaux blancs fondant à une température supérieure à 300°C et utilisé à l'état brut dans les synthèses ultérieures.

### Exemple 24

Une solution de 1,03 g de diphényl-7,7 (méthoxy-2 phényl)-4 phénylacétyl-2 perhydroisoindolol-4-(3aS,7aS) et de 0,95 g d'acide méthyl-4 phénylsulfonique dans 50 cm³ de toluène est portée au reflux pendant 3 heures. Après refroidissement, le mélange réactionnel est dilué par 200 cm³ de dichlorométhane, lavé par 100 cm³ d'une solution aqueuse de soude 4N (deux fois), par 100 cm³ d'eau, puis par 100 cm³ d'une solution aqueuse saturée en chlorure de sodium, séché sur sulfate de magnésium, et concentré à sec sous pression réduite (2,7 kPa). Le résidu est chromatographié sur colonne de gel de silice Merck (granulométrie 0,04-0,06 mm, diamètre 3,5 cm, hauteur 25 cm), en éluant sous une pression de 0,4 bar d'azote par un mélange de cyclohexane et d'acétate d'éthyle (50/50 en volumes) et en recueillant des fractions de 60 cm³. Les fractions 5 à 8 sont réunies et concentrées à sec sous pression réduite (2,7 kPa). Le résidu est trituré dans de l'éther de pétrole. On obtient 0,38 g de diphényl-4,4 (méthoxy-2 phényl)-7 phénylacétyl-2 hexahydro-2,3,3a,4,5,7a-(1H)isoindole-(3aS,7aR) sous la forme d'une meringue blanche.

Spectre RMN du proton (DMSO d₆): A température ambiante, on observe le mélange des rotamères: 2,5 à 3,9 (mt, -CH₂- et -CH<); 3,17 et 3,32 (ab J=12,5 et ab limite, -COCH₂Ar) ; 3,48 et 3,5 (2s, -OCH₃) ; 5,87 (mt, 1H, H vinylique); 6,7 à 7,6 (mt, 19H aromatiques).

Spectre infra-rouge (KBr), bandes caractéristiques (cm⁻¹): 3100-3000, 3000-2850, 2840, 1640, 1600, 1580, 1495, 1450, 1245, 1030, 750, 720, 700.

### Exemple 25

En opérant selon l'exemple 4, mais à partir de 1 g de chlorhydrate de diphényl-7,7 (méthoxy-2 phényl)-4 perhydroisoindolol-4-(3aS,4S,7aS) et de 0,37 g d'acide phénylacétique on obtient 0,3 g de diphényl-7,7 (méthoxy-2 phényl)-4 phénylacétyl-2 perhydroisoindolol-4-(3aS,4S,7aS) sous la forme de cristaux blancs fondant à 192°C.

### Exemple 26

En opérant selon l'exemple 4, mais à partir de 1 g de chlorhydrate de diphényl-7,7 (méthoxy-2 phényl)-4 perhydroisoindolol-4-(3aS,4S,7aS) et de 0,48 g d'acide (diméthylamino-2 phényl) acétique on obtient 0,91 g de diphényl-7,7 (méthoxy-2 phényl)-4 [(diméthylamino-2 phényl) acétyl]-2 perhydroisoindolol-4-(3aS,4S,7aS) sous la forme de cristaux blancs fondant à 207°C.

### Exemple 27

En opérant selon l'exemple 4, mais à partir de 1 g de chlorhydrate de diphényl-7,7 (méthoxy-2 phényl)-4 perhydroisoindolol-4-(3aS,4S,7aS) et de 0,48 g d'acide indényl-3 acétique on obtient 0,21 g de diphényl-7,7 (méthoxy-2 phényl)-4 [(indényl-3)-acétyl]-2 perhydroisoindolol-4-(3aS,4S,7aS) sous la forme d'une meringue blanche.

Spectre RMN du proton (DMSO d₆): A température ambiante, on observe le mélange des rotamères: 3,35 (2s, 3H du OCH₃); 4,82 (1H du OH); 6,1 et 6,4 (1H de l'indène); 6,8 à 7,6 (m, 18 H des phényles et de l'indène).

Spectre infra-rouge (KBr), bandes caractéristiques (cm⁻¹) : 3410, 3100-3000, 3000-2850, 2830, 1630, 1600, 1580, 1495, 1485, 1450, 1460-1425, 1235, 1025, 755, 755-700

### Exemple 28

En opérant selon l'exemple 4, mais à partir de 2 g de chlorhydrate de diphényl-7,7 (méthoxy-2 phényl)-4 perhydroisoindolol-4-(3aS,4S,7aS) et de 0,96 g d'acide indolyl-3 acétique on obtient 2,17 g de diphényl-7,7 (méthoxy-2 phényl)-4 [(indolyl-3)-acétyl)-2 perhydroisoindolol-4-(3aS,4S,7aS) sous la forme de cristaux blancs fondant à 142°C.

### Exemple 29

En opérant selon l'exemple 4, mais à partir de 0,8 g de chlorhydrate de diphényl-7,7 (méthoxy-2 phényl)-4 perhydroisoindolol-4-(3aS,4S, 7aS) et de 0,46 g d'acide (benzo[b]thiényl-3)acétique on obtient 0,5 g de diphényl-7,7 (méthoxy-2 phényl)-4 [(benzo[b]thiényl-3)acétyl]-2 perhydroisoindolol-4-(3aS,4S,7aS) sous la forme de cristaux blancs fondant à 130°C avec décomposition.

### Exemple 30

En opérant selon l'exemple 4, mais à partir de 0,73 g de chlorhydrate de diphényl-7,7 (méthoxy-2 phényl)-4 perhydroisoindolol-4-(3aS,4S,7aS) et de 0,5 g d'acide (benzyloxy-2 phényl)-2 propionique-(S) on obtient 0,73 g de diphényl-7,7 (méthoxy-2 phényl)-4 [(benzyloxy-2 phényl)-2 propionyl- (S)]-2 perhydroisoindolol-4-(3aS,4S,7aS) sous la forme d'une meringue blanche.

Spectre RMN du proton (DMSO d₆): A température ambiante, on observe le mélange des rotamères: 1,15 (2d, 3H du CH₃-CH); 3,35 (2s, 3H du OCH₃); 3,98 et 3,78 (2q, 1H du CH-CH₃); 4,2 (s, 1H du OH); 4,6 à 5 (2dd, 2H du CH₂O); 6,7 à 7,6 (m, 23H aromatiques).

A une solution de 0,73 g de diphényl-7,7 (méthoxy-2 phényl)-4 [(benzyloxy-2 phényl)-2 propionyl- (S)]-2 perhydroisoindolol-4-(3aS,4S,7aS) obtenus précédemment, dans 20 cm³ d'éthanol absolu, on ajoute 0,005 g de palladium sur charbon à 10% et on fait buller, à température ambiante de l'hydrogène dans le mélange réactionnel pendant 2 heures. Le mélange réactionnel est filtré sur célite, puis concentré à sec sous pression réduite (2,7 kPa). Le résidu est chromatographié sur colonne de gel de silice Merck (granulométrie 0,04-0,06 mm, diamètre 2,4 cm, hauteur 20 cm), en éluant sous une pression de 0,5 bar d'azote par un mélange de cyclohexane et d'acétate d'éthyle (80/20 en volumes) et en recueillant des fractions de 30 cm³. Les fractions 3 à 8 sont réunies et concentrées à sec sous pression réduite (2,7 kPa). On obtient 0,2 g de diphényl-7,7 (méthoxy-2 phényl) -4 [(hydroxy-2 phényl) -2 propionyl-(S)] -2 perhydroisoindolol-4-(3aS,4S,7aS) sous la forme de cristaux blancs fondant à 150°C avec décomposition.

L'acide (benzyloxy-2 phényl)-2 propionique-(S) peut être préparé de la manière suivante :

Une solution de 1,07 g de N-[(benzyloxy-2 phényl)-2-(S) propionyl] camphor-2,10-sultame-(1R,2S) dans un mélange de 0,47 cm³ de solution aqueuse de soude à 30% et de 10 cm³ de tétrahydrofurane est agitée à température ambiante pendant 48 heures. Le mélange réactionnel est concentré à sec sous pression réduite (2,7 kPa), puis dilué par 20 cm³ d'eau distillée, la phase aqueuse est extraite par 25 cm³ de dichlorométhane, puis acidifiée par 3 cm³ de solution aqueuse d'acide chlorhydrique à 37%, et enfin extraite 3 fois par 25 cm³ de dichlorométhane. La phase organique est séchée sur sulfate de magnésium et concentrée à sec sous pression réduite (2,7 kPa). On obtient 0,5 g d'acide (benzyloxy-2 phényl)-2 propionique-(S) sous la forme d'une huile incolore.

Spectre RMN du proton (CDCl₃) : 1,5 (d, 3H du CH₃-CH); 4,18 (q, 1H du CH-CH₃); 5,1 (2H du OCH₂); 6,95 à 7,45 (m, 9H aromatiques).

Le N-[(benzyloxy-2 phényl)-2-(S) propionyl]camphor-2,10-sultame-(1R,2S) peut être préparé de la manière suivante :

A une solution de 4,1 g de N-[(benzyloxy-2 phényl) acétyl] camphor2,10-sultame-(1R,2S) dans 40 cm³ de tétrahydrofurane refroidie à - 78°C on ajoute par fractions 1,62 g de tertiobutylate de potassium, puis on ajoute goutte à goutte à cette suspension une solution de 2,63 cm³ d'iodure de méthyle dans 2 cm³ de tétrahydrofurane. Le mélange réactionnel est agité à -78°C pendant 18 heures, puis on ajoute 40 cm³ d'une solution aqueuse saturée en chlorure d'ammonium. Après retour à température ambiante, le mélange réactionnel est extrait par 80 cm³ d'acétate d'éthyle, la phase organique est lavée deux fois par 25 cm³ d'une solution aqueuse saturée en chlorure d'ammonium, séchée sur sulfate de magnésium et concentrée à sec sous pression réduite (2,7 kPa). Le résidu est chromatographié sur colonne de gel de silice Merck (granulométrie 0,04-0,06 mm, diamètre 4 cm, hauteur 39 cm), en éluant sous une pression de 0,3 bar d'azote par du dichlorométhane et en recueillant des fractions de 25 cm³. Les fractions 21 à 53 sont réunies et concentrées à sec sous pression réduite (2,7 kPa). Le résidu est cristallisé dans l'oxyde de diisopropyle. On obtient 1,1 g de N-[(benzyloxy-2 phényl)-2-(S) propionyl] camphor-2,10-sultame-(1R,2S) sous la forme de cristaux blancs fondant à 131°C.

Le N-[(benzyloxy-2 phényl)-acétyl] camphor-2,10-sultame-(1R,2S) peut être préparé de la manière suivante :

A une solution refroidie à +10°C de 3,23 g de camphor-2,10-sultame-(1R,2S) dans 16 cm³ de dichlorométhane sec, on ajoute goutte à goutte une solution de 0,74 g de soude dans 20 cm³ d'eau distillée, puis 0,03 cm³ d'Aliquat 336®. On ajoute ensuite goutte à goutte à +10°C une solution de 3,9 g de chlorure de (méthoxy-2 phényl) acétyle dans 5 cm³ de dichlorométhane. Le mélange réactionnel est agité 1 heure à +10°C, décanté, la phase aqueuse est extraite par 80 cm³ de dichlorométhane, les phases organiques sont réunies, lavées par 80 cm³ d'eau distillée, séchées sur sulfate de magnésium et concentrées à sec sous pression réduite (2,7 kPa). Le résidu est cristallisé dans 15 cm³ d'oxyde de diisopropyle. On obtient 4,1 g de N-[(benzyloxy-2 phényl)acétyl] camphor-2,10-sultame-(1R,2S) sous la forme de cristaux blancs fondant à 116°C.

### Exemple 31

En opérant selon l'exemple 4, mais à partir de 12,27 g de chlorhydrate de diphényl-7,7 (méthoxy-2 phényl)-4 perhydroisoindolol-4 et de 7,88 g d'acide (benzyloxy-2 phényl)-acétique on obtient 16,4 g de diphényl-7,7 (méthoxy-2 phényl)-4 [(benzyloxy-2 phényl) acétyl]-2 perhydroisoindolol-4-(3aS,4S,7aS) sous la forme d'une meringue blanche.

Spectre RMN du proton (DMSO d₆): A température ambiante, on observe le mélange des rotamères: 3,4 (s, 3H du OCH₃); 5 (s, 2H du -CH₂O); 6,85 à 7,5 (m, 23H aromatiques).

En opérant selon l'exemple 30 à partir de 16,4 g de diphényl-7,7 (méthoxy-2 phényl)-4 [(benzyloxy-2 phényl)-2 acétyl]-2 perhydroisoindolol-4-(3aS,4S,7aS) on obtient 11,4 g de diphényl-7,7 (méthoxy-2 phényl)-4 [(hydroxy-2 phényl)-2 acétyl]-2 perhydroisoindolol-4-(3aS,4S,7aS) sous la forme de cristaux blancs fondant à 190°C.

### Exemple 32

A une suspension de 5 g de chlorhydrate de diphényl-4,4 (méthoxy-2 phényl)-7 hexahydro-2,3,3a,4,5,7a-(1H)-isoindole-(3aS,7aR) dans 70 cm³ de dichlorométhane sec on ajoute 0,16 g d'hydroxy-1 benzotriazole, 2,59 g d'acide (méthoxy-2 phényl)-2 propionique-(S), 2,08 cm³ de diisopropyléthylamine, puis on refroidit cette solution à +5°C et on ajoute rapidement une suspension de 2,8 g de chlorhydrate de (diméthylamino-3 propyl)-1 éthyl-3 carbodiimide dans 10 cm³ de dichlorométhane sec. Le mélange réactionnel est agité 2 heures à +5°C, puis 24 heures à température ambiante, lavé par 20 cm³ d'eau, puis par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 4 cm, hauteur 20 cm), en éluant sous une pression de 0,5 bar d'azote par un mélange de cyclohexane et d'acétate d'éthyle (80/20 en volumes) et en recueillant des fractions de 50 cm³. Les fractions 2 à 8 sont réunies et concentrées à sec sous pression réduite (2,7 kPa). Le résidu est trituré dans l'oxyde de diisopropyle. On obtient 4,43 g de diphényl4,4 (méthoxy-2 phényl)-7 [(méthoxy-2 phényl)-2 propionyl-(S)]-2 hexahydro-2,3,3a,4,5,7a-(1H)-isoindole-(3aS,7aR) sous la forme de cristaux blancs fondant à 104°C avec décomposition.

### Exemple 33

A une solution de 1,1 g de diphényl-7,7 (méthoxy-2 phényl)-4 perhydroisoindolediol-4,5-(3aS,4S,5S,7aS) et de 0,58 g d'acide (méthoxy-2 phényl)-2 propanoique-(S) dans 30 cm³ de dichlorométhane, refroidie à 0°C, on ajoute 30 mg d'hydrate d'hydroxy-1-benzotriazole, 0,66 g de (diméthylamino-3 propyl)-1 éthyl-3 carbodiimide et 0,6 cm³ de diisopropyléthylamine. On agite 3 heures et 30 minutes à température ambiante, ajoute 100 cm³ d'eau et 50 cm³ d'une solution aqueuse saturée de chlorure de sodium. La phase organique est décantée, séchée sur sulfate de magnésium et concentrée à sec sous pression réduite (2,7 kPa). Le résidu jaune obtenu est chromatographié sur colonne de gel de silice Merck (granulométrie 0,04-0,06 mm, diamètre 2 cm, hauteur 30 cm), en éluant sous une pression d'azote de 50 KPa par un mélange de cyclohexane et d'acétate d'éthyle (60/40 en volumes) et en recueillant des fractions de 25 cm³. Les fractions 6 à 14 sont réunies puis concentrées à sec sous pression réduite (2,7 kPa). On obtient 0,9 g de diphényl-7,7 (méthoxy-2 phényl)-4 [(méthoxy-2 phényl)-2 propionyl-S)]-2 perhydroisoindolediol-4,5 (3aS,4S,5S,7aS) fondant à 256°C.

Le diphényl-7,7 (méthoxy-2 phényl)-4 perhydroisoindolediol-4,5-(3aS,4S,5S,7aS) peut être obtenu de la manière suivante :

A 13 g de ditoluoyl-1,4-L-tartrate de diphényl-7,7 (méthoxy-2 phényl)-4 perhydroisoindolediol-4,5-(3aS,4S,5S,7aS) en solution dans 260 cm³ de méthanol, on ajoute 520 cm³ d'eau et 70 cm³ de soude aqueuse 1N; le mélange réactionnel est agité à température ambiante jusqu'à disparition du produit de départ. Les cristaux formés sont filtrés, essorés et séchés. On obtient 7,6 g de diphényl-7,7 (méthoxy-2 phényl)-4 perhydroisoindolediol-4,5-(3aS,4S,5S,7aS) fondant à 235°C.

Le ditoluoyl-1,4-L-tartrate de diphényl-7,7 (méthoxy-2 phényl)-4 perhydroisoindolediol-4,5-(3aS,4S,5S,7aS) peut être préparé de la manière suivante :

A une solution de 30 g de diphényl-7,7 (méthoxy-2 phényl)-4 perhydroisoindolediol-4,5-(3aRS,4RS,5RS,7aRS) dans 500 cm³ de méthanol, on ajoute, sous agitation, 29,2 g d'acide (-) ditoluoyl-1,4-L-tartrique.

Après dissolution totale, le mélange réactionnel est concentré à sec sous pression réduite (2,7 kPa); la meringue obtenue est cristallisée dans 500 cm³ d'oxyde d'éthyle. Les cristaux obtenus sont recristallisés à pouvoir rotatoire constant dans un mélange d'éthanol et d'eau (60/40 en volumes). On obtient 13,3 g de ditoluoyl-1,4-Ltartrate de diphényl-7,7 (méthoxy-2 phényl)-4 perhydroisoindolediol-4,5-(3aS,4S,5S,7aS) fondant à 240°C.

Le diphényl-7,7 (méthoxy-2 phényl)-4 perhydroisoindolediol-4,5-(3aRS,4RS,5RS,7aRS) peut être préparé de la manière suivante:

Un mélange de 2 g de benzyl-2 diphényl-7,7 (méthoxy-2 phényl)-4 perhydroisoindolediol-4,5(3aRS,4RS,5RS,7aRS) et de 50 cm³ d'éthanol est chauffé à 65°C sous agitation; on ajoute 0,65 g d'hydroxyde de palladium sur charbon à 20% puis le mélange réactionnel est hydrogéné, sous agitation, à une température de 65°C et sous pression atmosphérique. Aprés 1 heure de réaction, le volume théorique d'hydrogène a été absorbé; le mélange réactionnel est filtré, puis concentré à sec sous pression réduite (2,7 kPa). Le résidu est cristallisé dans 10 cm³ d'oxyde d'isopropyle. On obtient 1,45 g de diphényl-7,7 (méthoxy-2 phényl)-4 perhydroisoindolediol-4,5-(3aRS, 4RS,5RS,7aRS) fondant à 230°C.

Le benzyl-2 diphényl-7,7 (méthoxy-2 phényl)-4 perhydroisoindolediol-4,5-(3aRS,4RS,5RS,7aRS) peut être préparé de la manière suivante:

A une suspension de 84,4 g de bromure de méthoxy-2 phénylmagnésium dans 1000 cm³ de tétrahydrofurane, on ajoute goutte à goutte, à température ambiante, sous agitation, une solution de 22 g d'acétoxy-5 benzyl-2 diphényl-7,7 perhydroisoindolone-4-(3aRS,5RS,7aRS) dans 220 cm³ de tétrahydrofurane. Le mélange réactionnel est agité à température ambiante pendant 18 heures, traité par 200 cm³ d'une solution aqueuse saturée en chlorure d'ammonium, repris par 200 cm³ d'oxyde d'éthyle et 200 g de glace. La phase organique est décantée, séchée sur sulfate de magnésium, filtrée et concentrée à sec sous pression réduite (2,7 kPa). Le résidu est cristallisé dans 250 cm³ d'éther de pétrole puis recristallisé dans 200 cm³ de méthanol; les cristaux sont lavés par 200 cm³ d'oxyde d'isopropyle. On obtient 16,4 g de benzyl-2 diphényl-7,7 (méthoxy-2 phényl)-4 perhydroisoindolediol-4,5-(3aRS,4RS,5RS,7aRS) fondant à 236°C.

### Exemple 34

Le diphényl-7,7 (méthoxy-2 phényl)-4 [(benzyloxy-2 phényl) acétyl]-2 perhydroisoindolediol-4,5-(3aRS,4RS,5RS,7aRS) peut être préparé selon le mode opératoire de l'exemple 33, à partir de 1,24 g de diphényl7,7 (méthoxy-2 phényl) -4 perhydroisoindolediol-4,5-(3aRS,4RS,5RS, 7aRS) et de 0,8 g d'acide (benzyloxy-2) phényl acétique. On obtient 1,7 g de diphényl-7,7 (méthoxy-2 phényl)-4 [(benzyloxy-2 phényl) acétyl] -2 perhydroisoindolediol-4,5-(3aRS,4RS,5RS,7aRS) fondant à 158°C.

Un mélange de 1,5 g de diphényl-7,7 (méthoxy-2 phényl)-4 [(benzyloxy2 phényl) acétyl]-2 perhydroisoindolediol-4,5 (3aRS,4RS,5RS,7aRS) et de 50 cm³ d'éthanol est chauffé à 60°C sous agitation; on ajoute 0,5 g d'hydroxyde de palladium sur charbon à 20% puis le mélange réactionnel est hydrogéné, sous agitation, à une température de 60°C et sous pression atmosphérique. Aprés 45 minutes de réaction, le volume théorique d'hydrogène a été absorbé; le mélange réactionnel est filtré, puis concentré à sec sous pression réduite (2,7 kPa). Le résidu est cristallisé dans 20 cm³ d'oxyde d'isopropyle. On obtient 0,8 g de diphényl-7,7 (méthoxy-2 phényl)-4 [(hydroxy-2 phényl) acétyl]-2 perhydroisoindolediol-4,5-(3aRS,4RS,5RS,7aRS) fondant à 188-190°C.

### Exemple 35

En opérant selon le mode opératoire de l'exemple 21, à partir de 0,62 g de diphényl-7,7 (méthoxy-2 phényl)-4 perhydroisoindolediol-4,5-(3aRS,4RS,5RS,7aRS) et de 0,30 g d'acide (diméhylamino-2) phényl acétique, on obtient 0,47 g de diphényl-7,7 (méthoxy-2 phényl)-4 [(diméthylamino-2 phényl) acétyl]-2 perhydroisoindolediol-4,5-(3aRS,4RS,5RS,7aRS) fondant à 250°C.

### Exemple 36

En opérant selon le mode opératoire de l'exemple 21 à partir de 1,04 g de diphényl-7,7 (méthoxy-2 phényl)-4 perhydroisoindolediol-4,5-(3aS,4S,5S,7aS) et de 0,49 g d'acide indolyl-3 acétique, on obtient 1,25 g de diphényl-7,7 (méthoxy-2 phényl)-4 (indolyl-3 acétyl)-2 perhydroisoindolediol-4,5-(3aS,4S,5S,7aS) fondant à 210°C.

### Exemple 37

En opérant selon le mode opératoire de l'exemple 21 à partir de 0,62 g de diphényl-7,7 (méthoxy-2 phényl)-4 perhydroisoindolediol-4,5-(3aRS,4RS,5RS,7aRS) et de 0,31 g d'acide (N-méthyl indolyl)-3 acétique, on obtient 0,55 g de diphényl-7,7 (méthoxy-2 phényl)-4 [(N-méthyl indolyl-3) acétyl]-2 perhydroisoindolediol-4,5-(3aRS,4RS,5RS,7aRS) fondant à 240°C.

### Exemple 38

A une suspension de 6,4 g de diphényl-7,7 [(méthoxy-2 phényl)-2 propionyl-(S)]-2 perhydroisoindolone-4-(3aS,7aS) et de 8,7 g de chlorure de cérium anhydre dans 250 cm³ de tétrahydrofuranne anhydre, on ajoute goutte à goutte à température ambiante sous agitation une suspension de bromure de méthoxy-2 phénylmagnésium (préparée à partir de 14 cm³ de bromo-2 anisole et de 2,8 g de magnésium) dans 50 cm³ de tétrahydrofuranne anhydre. La suspension est agitée à température ambiante pendant 20 heures, puis portée au reflux pendant 6 heures. Après refroidissement, le mélange réactionnel est traité par une solution aqueuse saturée en chlorure d'ammonium, dilué par 500 cm³ d'acétate d'éthyle, lavé par 100 cm³ d'eau, puis par 100 cm³ d'une solution aqueuse saturée en chlorure de sodium (deux fois), séché sur sulfate de magnésium, filtré et concentré à sec sous pression réduite (2,7 kPa).Le résidu est chromatographié sur colonne de gel de silice Merck (granulométrie 0,04-0,06 mm, diamètre 5 cm, hauteur 40 cm), en éluant sous une pression de 0,5 bar d'azote par un mélange de cyclohexane et d'acétate d'éthyle (80/20 en volumes) et en recueillant des fractions de 100 cm³. Les fractions 16 à 26 sont réunies puis concentrées à sec sous pression réduite (2,7 kPa). Le résidu est cristallisé dans un mélange d'acétonitrile et d'oxyde de diisopropyle. On obtient 1,83 g de diphényl-7,7 (méthoxy-2 phényl)-4 [(méthoxy-2 phényl)-2 propionyl-(S)]-2 perhydroisoindolol-4-(3aS,4S,7aS) sous la forme de cristaux blancs fondant à 244°C.

La diphényl-7,7 [(méthoxy-2 phényl)-2 propionyl-(S)]-2 perhydroisoindo-lone-4-(3aS,7aS) peut être préparée de la manière suivante :

A une solution de 9,85 g de diphényl-7,7 perhydroisoindolone-4-(3aS,7aS) dans 120 cm³ de dichlorométhane sec on ajoute 0,46 g d'hydroxy-1 benzotriazole et 7,07 g d'acide (méthoxy-2 phényl)-2 propionique-(S), puis on refroidit cette solution à +5°C et on ajoute rapidement une suspension de 7,91 g de chlorhydrate de (diméthylamino-3 propyl)-1 éthyl-3 carbodiimide dans 130 cm³ de dichlorométhane sec. Le mélange réactionnel est agité 2 heures à +5°C, 2 heures à température ambiante, lavé par 50 cm³ d'eau, puis lavé par 50 cm³ d'une solution aqueuse saturée en chlorure de sodium (deux fois), séchée sur sulfate de magnésium, et concentré à sec sous pression réduite (2,7 kPa). Le résidu est chromatographié sur colonne de gel de silice Merck (granulométrie 0,04-0,06 mm, diamètre 6 cm, hauteur 30 cm), en éluant sous une pression de 0,5 bar d'azote par un mélange de cyclohexane et d'acétate d'éthyle (80/20 en volumes) et en recueillant des fractions de 50 cm³. Les fractions 36 à 60 sont réunies et concentrées à sec sous pression réduite (2,7 kPa). On obtient 12,3 g de diphényl-7,7 [(méthoxy-2 phényl)-2 propionyl-(S)]-2 perhydroisoindolone-4-(3aS,7aS) sous forme de solide blanc.

Spectre RMN du proton (DMSO d₆) : A température ambiante on observe le mélange des deux rotamères: 1,13 et 1,23 (2d, J=7,5, 3H en totalité, _CH₃); 1,9 à 2,3 (mt, 2H, -CH₂- en 5); 2,6 à 4,2 (mt, -CH₂- et -CH); 3,36 et 3,86 (2s, 3H en totalité, -OCH₃); 6,7 à 7,7 (mt, 14H, aromatiques).

### Exemple 39

A une suspension de 4,09 g de diphényl-7,7 phénylacétyl-2 perhydroisoindolone-4-(3aRS,7aRS) dans 50 cm³ de tétrahydrofuranne, on ajoute une solution de bromure de phénylmagnésium (préparée à partir de 3,14 g de bromobenzène et 0,48 de magnésium dans 30 cm³ d'éther). Le mélange réactionnel est agité 1 heure à 25°C puis chauffé au reflux pendant 2,5 heures, refroidi et traité par 150 cm³ de solution saturée de chlorure d'ammonium. La phase organique est lavée à l'eau (2 x 100 cm³). Les phases aqueuses sont extraites par 100 cm³ d'acétate d'éthyle. Les phases organiques réunies sont lavées par 100 cm³ de solution saturée de chlorure de sodium, séchées sur sulfate de magnésium et concentrées à sec sous pression réduite (2,7 kPa). Le résidu est cristallisé dans 30 cm³ d'acétonitrile pour donner 2,9 g de phénylacétyl-2 triphényl-4,7,7 perhydroisoindolol-4-(3aRS,4RS,7aRS) sous la forme de cristaux blancs fondant à 270°C.

### Exemple 40

A une suspension de bromure de phénylmagnésium (préparé à partir de 4,7 cm³ de bromobenzène et 1,1 g de magnésium) dans 40 cm³ d'éther éthylique, on ajoute goutte à goutte à 5°C pendant 20 minutes une solution de 7,0 g d'(indolyl-3 acétyl)-2 diphényl-7,7 perhydroisoindolone-4-(3aRS,7aRS) dans 50 cm³ de tétrahydrofuranne. Le mélange réactionnel est agité à température ambiante pendant 4 heures, traité par 100 cm³ d'une solution aqueuse saturée en chlorure d'ammonium, extrait avec trois fois 150 cm³ d'acétate d'éthyle. La solution organique est lavée avec 200 cm³ d'une solution aqueuse saturée en chlorure de sodium, séchée sur sulfate de magnésium, filtrée et concentrée à sec sous pression réduite (2,7 kPa). Le résidu est chromatographié sur colonne de gel de silice Merck (granulométrie 0,06-0,20 mm, diamètre 4 cm, hauteur 30 cm) en éluant sous une pression de 0,5 bar d'azote par un mélange de cyclohexane et d'acétate d'éthyle (60/40 en volume) et en recueillant des fractions de 100 cm³. Après concentration sous pression réduite (2,7 kPa) on obtient 6,6 g d'(indolyl-3 acétyl)-2 triphényl-4,7,7 perhydroisoindolol-4-(3aRS,4RS,7aRS) sous forme de solide blanc fondant à 187°C.

La présente invention concerne également les compositions pharmaceutiques constituées par un produit de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, sublinguale, rectale, topique, oculaire, intranasale ou en aérosols à visée pulmonaire.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

Les compositions pour administration oculaires peuvent être des instillations.

Les compositions pour administration intranasale peuvent être des solutions ou des poudres pharmaceutiquement acceptables destinées à des gouttes ou à des pulvérisations.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, du sérum ou tout autre véhicule pharmaceutiquement acceptable. Pour l'usage sous forme d'aérosols secs destinés à être directement inhalés, le principe actif est est finement divisé et associé à un diluant ou véhicule solide hydrosoluble d'une granulométrie de 30 à 80 µm, par exemple le dextrane, le mannitol ou le lactose.

En thérapeutique humaine, les produits selon l'invention peuvent être particulièrement utiles dans le traitement des douleurs d'origine traumatique, post-chirurgicale, menstruelle, céphaliques, dans les algies vasculaires de la face (cluster headache) et dans les traitements des migraines. Les nouveaux dérivés de l'isoindole sont également utiles dans le traitement de l'inflammation en rhumathologie, dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses, les brûlures et dans les troubles inflammatoires dentaires ou oculaires et dans le domaine des sécretions lachrymales; ils sont également utiles dans les traitements des manifestations spasmodiques, douloureuses et inflammatoires des voies digestives (colites ulcéreuses, syndrome du colon irritable, maladie de Crohn), des voies urinaires (hyperréflexies urinaires, cystites) et des voies respiratoires (asthme, hypersécretion bronchique, bronchite chronique, rhinites). Les produits selon l'invention peuvent également trouver une application dans les traitements de maladies neurologiques, maladie de Parkinson, maladie d'Alzeimer, dans les traitements des maladies inflammatoires et/ou autoimmunes et/ou démyélinisantes du système nerveux central et/ou périphérique (sclérose en plaque, syndrome de Guillain-Barré, encéphalopathies d'origine virale ...), dans les syndromes neurologiques en relation avec une extravasation plasmatique (oedème de la moelle épinière, oedème cérébral...), en relation avec une atteinte de la barrière hématoencéphalique ou dans tout syndrome neurologique spastique (traitements myorelaxants). Les produits selon l'invention peuvent également être utiles dans les traitements de l'anxiété, des psychoses, de la schizophrénie, ou encore dans les traitements des troubles cardiovasculaires tels de l'hypotension. Une autre application peut être également le traitement des troubles gynécologiques, le traitement des troubles liés à une mauvaise régulation de la croissance (nanisme, hypothrophies secondaires à des maladies chroniques de l'enfant, ostéoporose, développement de greffes).

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

### Exemple

On prépare, selon la technique habituelle, des comprimés de produit actif ayant la composition suivante :
- diphényl-7,7 (méthoxy-2 phényl)-4 [(méthoxy-2 phényl)-2 propionyl-(S)]-2 perhydroisoindolol-4-(3aS,4S,7aS) 25 mg
- amidon 83 mg
- silice 30 mg
- stéarate de magnésium 3 mg

## Revendications

1. Un dérivé de perhydroisoindole caractérisé en ce qu'il répond à la formule générale : 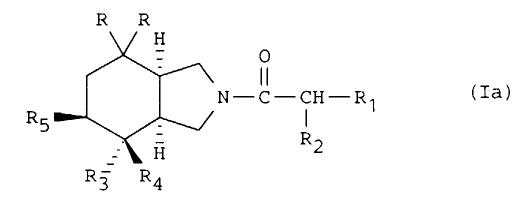 dans laquelle
- les symboles R sont identiques et représentent des radicaux phényle éventuellement substitués par un atome d'halogène ou par un radical méthyle en position 2 ou 3,
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle, indényle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle ou alcoyloxy,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino,
- le symbole R₃ représente un radical phényle éventuellement substitué en position -2 par un radical alcoyle ou alcoyloxy contenant 1 ou 2 atomes de carbone, et
- le symbole R₄ représente un atome de fluor ou un radical hydroxy, et le symbole R₅ représente un atome d'hydrogène ou bien
- les symboles R₄ et R₅ représentent des radicaux hydroxy, ou bien
- le symbole R₄ forme avec R₅ une liaison,
étant entendu que les radicaux alcoyle et acyle sont (sauf mention spéciale) droits ou ramifiés et contiennent 1 à 4 atomes de carbone, et étant entendu que la chaîne -CHR₁R₂ peut exister sous ses formes (R) et (S) ou leurs mélanges,
ainsi que la forme racémique du dérivé de formule générale (Ia), et les mélanges avec la forme racémique,
ainsi que ses sels lorsqu'ils existent.

2. Un dérivé de perhydroisoindole selon la revendication 1, caractérisé en ce que le symbole R₁ est un radical hétérocyclyle mono ou polycyclique, saturé ou insaturé choisi parmi thiényle, furyle, pyridyle, dithiinyle, indolyle, isoindolyle, benzothiényle, thiazolyle, isothiazolyle, oxazolyle, imidazolyle, pyrrolyle, triazolyle, thiadiazolyle, quinolyle, isoquinolyle, ou naphtyridinyle.

3. Un dérivé de perhydroisoindole selon la revendication 1, caractérisé en ce que :
- le symbole R₁ représente un radical phényle éventuellement substitué par un ou plusieurs radicaux hydroxy, alcoyloxy, dialcoylamino, naphtyle, indényle ou hétérocyclyle mono ou polycyclique choisi parmi thiényle, indolyle, benzothiényle et éventuellement substitué par un atome d'halogène ou par un radical alcoyle ou alcoyloxy,
- le symbole R₂ représente un atome d'hydrogène ou un radical hydroxy, alcoyle ou amino,
- le symbole R₃ représente un radical phényle éventuellement substitué en position -2 par un radical alcoyle ou alcoyloxy contenant 1 ou 2 atomes de carbone, et
- le symbole R₄ représente un atome de fluor ou un radical hydroxy, et le symbole R₅ représente un atome d'hydrogène ou bien
- les symboles R₄ et R₅ représentent des radicaux hydroxy, ou bien
- le symbole R₄ forme avec R₅ une liaison.

4. Un dérivé de perhydroisoindole selon la revendication 1, caractérisé en ce qu'il s'agit du diphényl-7,7 (méthoxy-2 phényl)-4 [(méthoxy-2 phényl)-2 propionyl]-2 perhydroisoindolol-4; du diphényl-7,7 (méthoxy-2 phényl)-4 [(méthoxy-2 phényl)-2 propionyl]-2 perhydroisoindolediol-4,5; du diphényl-7,7 (méthoxy-2 phényl)-4 [(hydroxy-2 phényl)-2 acétyl]-2 perhydroisoindolol-4; du diphényl-7,7 (méthoxy-2 phényl)-4 (indolyl-3 acétyl)-2 perhydroisoindolediol-4,5; du diphényl-7,7 (méthoxy-2 phényl)-4 [(méthoxy-2 phényl)-acétyl]-2 perhydroisoindolol-4; ou du diphényl-7,7 (méthoxy-2 phényl)-4 [(hydroxy-2 phényl)-2 propionyl]-2 perhydroisoindolol-4.

5. Procédé de préparation d'un nouveau dérivé de perhydroisoindole selon la revendication 1, caractérisé en ce que l'on fait agir un acide ou un dérivé réactif de l'acide de formule générale : 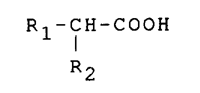 dans laquelle R₁ et R₂ sont définis comme dans la revendication 1, sur un dérivé de l'isoindole de formule générale : 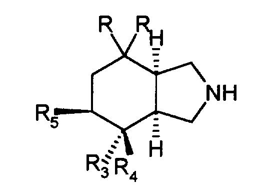 dans laquelle les symboles R, R₃, R₄ et R₅ sont définis comme dans la revendication 1, puis transforme éventuellement le produit obtenu pour lequel R₄ est un radical hydroxy et R₅ est un atome d'hydrogène en un produit pour lequel R₄ est un atome de fluor et R₅ est un atome d'hydrogène ou pour lequel R₄ et R₅ forment ensemble une liaison, et transforme éventuellement le produit obtenu en un sel lorsqu'ils existent.

6. Procédé de préparation d'un dérivé de perhydroisoindole selon la revendication 1 pour lequel R₅ est autre qu'un radical hydroxy, caractérisé en ce que l'on fait agir un compose organométallique de formule générale :
R₃-M
dans laquelle R₃ est defini comme précédemment, et M représente le lithium, un radical MgX ou CeX₂ pour lequel X est un atome d'halogene, sur un derivé de perhydroisoindolone de formule générale : 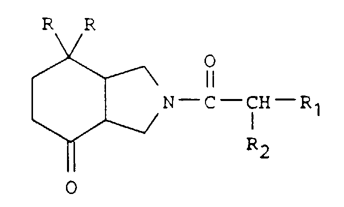 dans laquelle R, R₁ et R₂ sont définis comme dans la revendication 1, puis transforme éventuellement l'alcool obtenu en un dérivé de perhydroisoindole pour lequel R₄ est un atome de fluor et R₅ est un atome d'hydrogène ou en un dérivé de perhydroisoindole pour lequel R₄ et R₅ forment ensemble une liaison, et sépare éventuellement les isomères, et/ou transforme le produit obtenu en un sel, lorsqu'ils existent.

7. Composition pharmaceutique caractérisée en ce qu'elle contient au moins un produit selon la revendication 1, à l'état pur ou sous forme d'association avec un ou plusieurs adjuvants ou diluants compatibles et pharmaceutiquement acceptables.

## Claims

1. A perhydroisoindole derivative, characterized in that it corresponds to the general formula: 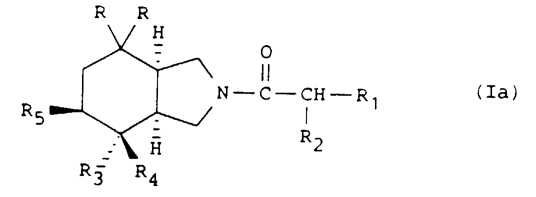 in which
- the symbols R are identical and represent phenyl radicals which are optionally substituted by a halogen atom or by a methyl radical in position 2 or 3,
- the symbol R₁ represents a phenyl radical which is optionally substituted by one or more halogen atoms or hydroxyl radicals, alkyl radicals which may be optionally substituted (by halogen atoms or amino, alkylamino or dialkylamino radicals), alkyloxy radicals or alkylthio radicals which may be optionally substituted [by hydroxyl radicals, amino radicals, alkylamino radicals or dialkylamino radicals which are optionally substituted (by phenyl, hydroxyl or amino radicals), or dialkylamino radicals, the alkyl parts of which, with the nitrogen atom to which they are attached, form a heterocycle having 5 to 6 chain members which may contain another heteroatom chosen from oxygen, sulphur or nitrogen and optionally substituted by an alkyl, hydroxyl or hydroxyalkyl radical)], or is substituted by amino radicals, alkylamino radicals or dialkylamino radicals, the alkyl parts of which, with the nitrogen atom to which they are attached, may form a heterocycle as defined above, or represents a cyclohexadienyl radical, naphthyl radical, indenyl radical or mono- or polycyclic, saturated or unsaturated, heterocyclyl radical containing 5 to 9 carbon atoms and one or more heteroatoms chosen from oxygen, nitrogen or sulphur, which is optionally substituted by a halogen atom or by an alkyl or alkyloxy radical,
- the symbol R₂ represents a hydrogen or halogen atom or a hydroxyl, alkyl, aminoalkyl, alkylaminoalkyl, dialkylaminoalkyl, alkyloxy, alkylthio, acyloxy, carboxyl, alkyloxycarbonyl, dialkylaminoalkyloxycarbonyl, benzyloxycarbonyl, amino or acylamino radical,
- the symbol R₃ represents a phenyl radical which is optionally substituted in position 2 by an alkyl or alkyloxy radical containing 1 or 2 carbon atoms and
- the symbol R₄ represents a fluorine atom or a hydroxyl radical and the symbol R₅ represents a hydrogen atom, or
- the symbols R₄ and R₅ represent hydroxyl radicals, or
- the symbol R₄ forms a bond with R₅,
on the understanding that the alkyl and acyl radicals mentioned above are (unless mentioned specifically) straight or branched and contain 1 to 4 carbon atoms, and on the understanding that the chain -CHR₁R₂ can exist in its (R) and (S) forms or their mixtures,
and the racemic form of the derivative of general formula (Ia), and the mixtures with the racemic form,
and its salts, where they exist.

2. A perhydroisoindole derivative according to claim 1, characterized in that the symbol R₁ is a mono- or polycyclic, saturated or unsaturated, heterocyclyl radical chosen from thienyl, furyl, pyridyl, dithiinyl, indolyl, isoindol, benzothienyl, thiazolyl, isothiazolyl, oxazolyl, imidazolyl, pyrrolyl, triazolyl, thiadiazolyl, quinolyl, isoquinolyl and naphthyridinyl.

3. A perhydroisoindole derivative according to claim 1, characterized in that:
- the symbol R₁ represents a phenyl radical which is optionally substituted by one or more hydroxyl, alkyloxy or dialkylamino radicals, naphthyl radical, indenyl radical or mono- or polycyclic heterocyclyl radical chosen from thienyl, indolyl and benzothienyl and optionally substituted by a halogen atom or by an alkyl or alkyloxy radical,
- the symbol R₂ represents a hydrogen atom or a hydroxyl, alkyl or amino radical,
- the symbol R₃ represents a phenyl radical which is optionally substituted in position 2 by an alkyl or alkyloxy radical containing 1 or 2 carbon atoms and
- the symbol R₄ represents a fluorine atom or a hydroxyl radical and the symbol R₅ represents a hydrogen atom, or
- the symbols R₄ and R₅ represent hydroxyl radicals, or
- the symbol R₄ forms a bond with R₅.

4. A perhydroisoindole derivative according to claim 1, characterized in that it is 7,7-diphenyl-4(2-methoxyphenyl) -2 -[2 -(2-methoxyphenyl)propionyl]perhydroisoindol-4-ol; 7,7-diphenyl-4- (2-methoxyphenyl)2 - [2- (2-methoxyphenyl)propionyl] -perhydroisoindole-4,5diol; 7,7-diphenyl-4-(2-methoxyphenyl)-2-[2-(2-hydroxyphenyl)acetyl] -perhydroisoindol-4-ol; 7,7-diphenyl-4- (2methoxyphenyl)-2- (3-indolylacetyl)perhydroisoindole-4,5diol; 7,7-diphenyl-4- (2-methoxyphenyl) -2-[(2-methoxyphenyl) acetyl] perhydroisoindol-4-ol; or 7,7-diphenyl-4(2-methoxyphenyl)-2-[2-(2-hydroxyphenyl)propionyl] perhydroisoindol-4-ol.

5. Process for the preparation of a new perhydroisoindole derivative according to claim 1, characterized in that an acid or a reactive derivative of the acid of the general formula: 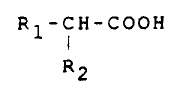 in which R₁ and R₂ are as defined in claim 1, is allowed to act on an isoindole derivative of the general formula: 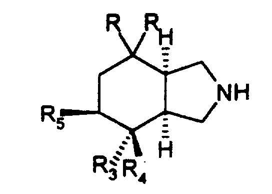 in which the symbols R, R₃, R₄ and R₅ are as defined in claim 1, if appropriate the product obtained in which R₄ is a hydroxyl radical and R₅ is a hydrogen atom is subsequently converted into a product in which R₄ is a fluorine atom and R₅ is a hydrogen atom or in which R₄ and R₅ together form a bond, and if appropriate the product obtained is converted into a salt, where these exist.

6. Process for the preparation of a perhydroisoindole derivative according to claim 1 in which R₅ is other than a hydroxyl radical, characterized in that an organometallic compound of the general formula:
R₃-M
in which R₃ is as defined above and M represents lithium or a CeX₂ or MgX radical, in which X is a halogen atom, is allowed to act on a perhydroisoindolone derivative of the general formula: 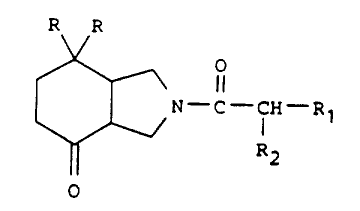 in which R, R₁ and R₂ are as defined in claim 1, if appropriate the alcohol obtained is subsequently converted into a perhydroisoindole derivative in which R₄ is a fluorine atom and R₅ is a hydrogen atom or into a perhydroisoindole derivative in which R₄ and R₅ together form a bond, and if appropriate the isomers are separated, and/or the product obtained is converted into a salt, where these exist.

7. Pharmaceutical composition, characterized in that it contains at least one product according to claim 1 in the pure state or in the form of a combination with one or more compatible and pharmaceutically acceptable adjuvants or diluents.

## Patentansprüche

1. Ein Perhydroisoindol-Derivat, dadurch gekennzeichnet, daß es der allgemeinen Formel: 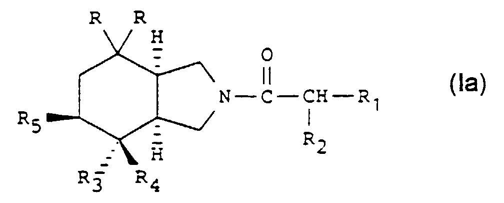 entspricht, in der
- die Symbole R gleich sind und Phenylreste darstellen, die gegebenenfalls mit einem Halogenatom oder mit einem Methylrest in 2- oder 3-Position substituiert sind,
- das Symbol R₁ einen Phenylrest darstellt, der gegebenenfalls mit einem oder mehreren Halogenatomen oder Hydroxyresten, Alkylresten, die gegebenenfalls substituiert sein können (mit Halogenatomen oder Amino-, Alkylamino- oder Dialkylaminoresten), Alkoxy- oder Alkylthioresten, die gegebenenfalls substituiert sein können [mit Hydroxy-, Amino-, Alkylamino- oder Dialkylaminoresten, die gegebenenfalls substituiert sind (mit Phenyl-, Hydroxy- oder Aminoresten), oder Dialkylaminoresten, deren Alkylteile mit dem Stickstoffatom, mit dem sie verknüpft sind, einen Heterocyclus mit 5 bis 6 Kettengliedern bilden, der ein anderes unter Sauerstoff, Schwefel oder Stickstoff ausgewähltes Heteroatom enthalten kann, der gegebenenfalls substituiert ist (mit einem Alkyl-, Hydroxy- oder Hydroxyalkylrest)], substituiert ist oder mit Amino-, Alkylamino- oder Dialkylaminoresten, deren Alkylteile mit dem Stickstoffatom, mit dem sie verknüpft sind, einen wie oben definierten Heterocyclus bilden können, substituiert ist, oder einen Cyclohexadienyl-, Naphthyl-, Indenylrest oder einen mono- oder polycyclischen, gesättigten oder ungesättigten heterocyclischen Rest darstellt, der 5 bis 9 Kohlenstoffatome und ein oder mehrere unter Sauerstoff, Stickstoff oder Schwefel ausgewählte Heteroatome enthält und der gegebenenfalls mit einem Halogenatom oder mit einem Alkyl- oder Alkoxyrest substituiert ist,
- das Symbol R₂ ein Wasserstoff- oder Halogenatom oder einen Hydroxy-, Alkyl-, Aminoalkyl-, Alkylaminoalkyl-, Dialkylaminoalkyl-, Alkoxy-, Alkylthio-, Acyloxy-, Carboxy-, Alkoxycarbonyl-, Dialkylaminoalkoxycarbonyl-, Benzyloxycarbonyl-, Amino- oder Acylaminorest darstellt,
- das Symbol R₃ einen Phenylrest darstellt, der gegebenenfalls in 2-Position mit einem Alkyl- oder Alkoxyrest mit 1 oder 2 Kohlenstoffatomen substituiert ist, und
- das Symbol R₄ ein Fluoratom oder einen Hydroxyrest darstellt und das Symbol R₅ ein Wasserstoffatom darstellt oder aber
- die Symbole R₄ und R₅ Hydroxyreste darstellen oder aber
- das Symbol R₄ mit R₅ eine Bindung bildet,
wobei es sich versteht, daß die Alkyl- und Acylreste (außer bei spezieller Erwähnung) gerade oder verzweigt sind und 1 bis 4 Kohlenstoffatome enthalten, und wobei es sich versteht, daß die Kette -CHR₁R₂ in ihren (R)- und (S)-Formen oder deren Gemischen existieren kann,
sowie die Racematform des Derivats der allgemeinen Formel (la) und die Gemische mit der Racematform
sowie seine Salze, wenn sie existieren.

2. Ein Perhydroisoindol-Derivat gemäß Anspruch 1, dadurch gekennzeichnet, daß das Symbol R₁ ein mono- oder polycyclischer gesättigter oder ungesättigter heterocyclischer Rest ist, der unter einem Thienyl-, Furyl-, Pyridyl-, Dithiinyl-, Indolyl-, Isoindolyl-, Benzothienyl-, Thiazolyl-, Isothiazolyl-, Oxazolyl-, Imidazolyl-, Pyrrolyl-, Triazolyl-, Thiadiazolyl-, Chinolyl-, Isochinolyl- oder Naphthyridinylrest ausgewählt ist.

3. Ein Perhydroisoindol-Derivat gemäß Anspruch 1 , dadurch gekennzeichnet, daß:
- das Symbol R₁ einen Phenylrest darstellt, der gegebenenfalls mit einem oder mehreren Hydroxy-, Alkoxy-, Dialkylamino-, Naphthyl-, Indenylresten oder einem mono- oder polycyclischen heterocyclischen Rest substituiert ist, der unter einem Thienyl-, Indolyl- und Benzothienylrest ausgewählt ist und gegebenenfalls mit einem Halogenatom oder einem Alkyl- oder Alkoxyrest substituiert ist,
- das Symbol R₂ ein Wasserstoffatom oder einen Hydroxy-, Alkyl- oder Aminorest darstellt,
- das Symbol R₃ einen Phenylrest darstellt, der gegebenenfalls in 2-Position mit einem Alkyl- oder Alkoxyrest mit 1 oder 2 Kohlenstoffatomen substituiert ist, und
- das Symbol R₄ ein Fluoratom oder einen Hydroxyrest darstellt und das Symbol R₅ ein Wasserstoffatom darstellt oder aber
- die Symbole R₄ und R₅ Hydroxyreste darstellen oder aber
- das Symbol R₄ mit R₅ eine Bindung bildet.

4. Ein Perhydroisoindol-Derivat gemäß Anspruch 1, dadurch gekennzeichnet, daß es sich um 7,7-Diphenyl-4-(2-methoxyphenyl)-2-[2-(2-methoxyphenyl)propionyl]perhydroisoindol-4-ol; 7,7-Diphenyl-4-(2-methoxyphenyl)-2-[2-(2-methoxyphenyl)propionyl]perhydroisoindol-4,5-diol; 7,7-Diphenyl-4-(2-methoxyphenyl)-2[2-(2-hydroxyphenyl)acetyl]perhydroisoindol-4-ol; 7,7-Diphenyl-4-(2-methoxyphenyl)2-(3-indolylacetyl)perhydroisoindol-4,5-diol; 7,7-Diphenyl-4-(2-methoxyphenyl)2-[(2-methoxyphenyl)acetyl]perhydroisoindol-4-o1 oder 7,7-Diphenyl-4-(2-methoxyphenyl)-2-[2-(2-hydroxyphenyl)propionyl]perhydroisoindol-4-ol handelt.

5. Verfahren zur Herstellung eines neuen Perhydroisoindol-Derivats gemäß Anspruch 1, dadurch gekennzeichnet, daß man eine Säure oder ein reaktives Säurederivat der allgemeinen Formel: 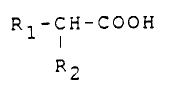 in der R₁ und R₂ wie in Anspruch 1 definiert sind, auf ein Derivat des Isoindols der allgemeinen Formel: 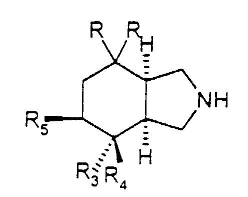 in der die Symbole R, R₃, R₄ und R₅ wie in Anspruch 1 definiert sind, einwirken läßt, dann gegebenenfalls das erhaltene Produkt, für das R₄ ein Hydroxyrest ist und R₅ ein Wasserstoffatom ist, in ein Produkt überführt, für das R₄ ein Fluoratom ist und R₅ ein Wasserstoffatom ist oder für das R₄ und R₅ zusammen eine Bindung bilden, und gegebenenfalls das erhaltene Produkt in ein Salz überführt, wenn sie existieren.

6. Verfahren zur Herstellung eines Perhydroisoindol-Derivats gemäß Anspruch 1, für das R₅ anders als ein Hydroxyrest ist, dadurch gekennzeichnet, daß man eine organometallische Verbindung der allgemeinen Formel:
R₃-M
in der R₃ wie vorhergehend definiert ist und M Lithium, einen MgX- oder CeX₂-Rest, für den X ein Halogenatom ist, darstellt, auf ein Perhydroisoindolon-Derivat der allgemeinen Formel: 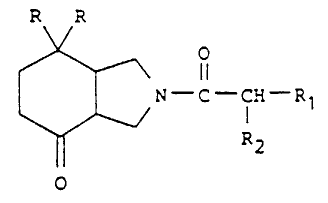 in der R, R₁ und R₂ wie in Anspruch 1 definiert sind, einwirken läßt, dann gegebenenfalls den erhaltenen Alkohol in ein Perhydroisoindol-Derivat, für das R₄ ein Fluoratom ist und R₅ ein Wasserstoffatom ist, oder in ein Perhydroisoindol-Derivat, für das R₄ und R₅ zusammen eine Bindung bilden, überführt und gegebenenfalls die Isomere trennt und/oder das erhaltene Produkt in ein Salz überführt, wenn sie existieren.

7. Pharmazeutische Zusammensetzung, dadurch gekennzeichnet, daß sie wenigstens ein Produkt gemäß Anspruch 1 in reinem Zustand oder in Form einer Vereinigung mit einem oder mehreren kompatiblen und pharmazeutisch annehmbaren Hilfsstoffen oder Verdünnungsmitteln enthält.
